# EUROPEAN PATENT SPECIFICATION

(11) **EP 1 874 407 B1**
(45) Date of publication and mention of the grant of the patent: **09.09.2015**
(21) Application number: 06742586.8
(22) Date of filing: 13.04.2006
(51) Int. Cl.: A61P 35/00

(54) **SUBSTANCES AND PHARMACEUTICAL COMPOSITIONS FOR THE INHIBITION OF GLYOXALASES AND THEIR USE AS ANTI-FUNGAL AGENTS**
SUBSTANZEN UND PHARMAZEUTISCHE ZUSAMMENSETZUNGEN FÜR DIE HEMMUNG VON GLYOXALASEN UND IHRE VERWENDUNG ALS ANTIFUNGALE MITTEL
SUBSTANCES ET COMPOSITIONS PHARMACEUTIQUES DESTINEES A INHIBER LES GLYOXALASES ET LEUR UTILISATION COMME AGENTS ANTIFONGIQUES

(30) Priority: 15.04.2005 DE 102005018641; 15.04.2005 DE 102005018642
(43) Date of publication of application: 09.01.2008
(73) Proprietor: Biomac Privatinstitut für medizinische und Zahnmedizinische Forschung, Entwicklung und Diagnostik GmbH, 04103 Leipzig (DE)
(72) Inventor: HUSE, Klaus, 04416 Markkleeberg (DE); BIRKENMEIER, Gerd, 04103 Leipzig (DE); BIRKENMEIER, Monika, 04103 Leipzig (DE)
(74) Representative: Kailuweit & Uhlemann Patentanwälte Partnerschaft mbB
(86) International application number: PCT/EP2006/003467
(87) International publication number: WO 2006/108682

(56) References cited:
- EP-A- 0 273 202
- EP-A- 0 366 632
- WO-A-01/68085
- WO-A-03/088955
- WO-A-2006/099325
- JP-A- 8 325 107
- US-A- 5 633 285
- CARBALLEIRA NÉSTOR M ET AL: "TOTAL SYNTHESIS AND IN VITRO-ANTIFUNGAL ACTIVITY OF (+/-)-2-METHOXYTETRADECANOIC ACID" ARCHIV DER PHARMAZIE, VCH VERLAGSGESELLSCHAFT MBH, WEINHEIM, DE, vol. 337, no. 3, 2004, pages 152-155, XP008070403 ISSN: 0365-6233
- HALL S S ET AL: "Synthesis and evaluation of alpha-hydroxythiol esters as antitumor agents and glyoxalase I inhibitors." JOURNAL OF MEDICINAL CHEMISTRY. OCT 1977, vol. 20, no. 10, October 1977 (1977-10), pages 1239-1242, XP002405487 ISSN: 0022-2623
- PATENT ABSTRACTS OF JAPAN vol. 008, no. 056 (C-214), 14 March 1984 (1984-03-14) & JP 58 213716 A (JIYUNICHI IWAMURA), 12 December 1983 (1983-12-12)

## Description

### FIELD OF THE INVENTION

The invention relates to to pharmaceutical compositions comprising one or more compounds according to formula (I) and glucose for the use in the treatment and/or prohylaxis of a fungal infection.

### BACKGROUND OF THE INVENTION

A multitude of fungi which do not cause any problems live on and partially in the body of all human beings and animals. About 100 of the more than 100.000 species of fungi described so far are known to be capable of causing disease in human beings and animals. Moreover, many fungi are known to infect and damage plants.

Fungal infections, in particular when they remain superficial and locally circumscribed, such as e.g. athletes' foot, can be relatively benign. Nevertheless, such infections can cause discomfort and require treatment.

Systemic fungal infections are life threatening diseases that are difficult to treat with available medication.

Many fungal infections (mycosis) are the result of an opportunistic infection on the basis of a weakened immune defence. The weakening of the immune defence is often the result of chemotherapy, an organ transplantation accompanied by immune suppression or an HIV infection. In such situations, fungi populating the mucosa (mucosal fungi) can enter the blood or vessels and thus can become highly dangerous for the patient (systemic mycosis). The most common fungi that are pathogenic to human beings are species of Candida and Aspergillus.

In Germany alone, about 40.000 people are affected by an invasive Candida infection each year. Amongst hospital infections this yeast is in the meanwhile ranked fourth amongst the most dangerous pathogens and necessitates high costs for therapy. Antimycotic therapy also has a high significance for the reduction of candidiasis and aspergillosis in case of transplantations (Higashiyama and Kohno, 2004). Fungal infections represent the main cause of death of recipients of bone marrow transplantation. About half of all recipients of transplants fall ill with a systemic fungal infection and about one third dies due to a non controllable infection (Epstein et al., 2003).

Candida belongs to yeasts. The transmittal of yeasts is possible via contaminated food but also by direct contact with other human beings and animals, particularly pets. Besides the affection of the mucosa in mouth, esophagus or vagina, a Candida infection can also result in a spread into liver, kidney, spleen and other organs. Candida albicans is the most commonly found species, but lately Candida glabrata and Candida krusei are found more often in isolates, often exhibiting resistance against common antifungal agents.

Apart from infections by Candida, Aspergillus spp. (moulds), in particular Aspergillus fumigatus, can cause the life threatening disease invasive aspergillosis. The fungus grows from the lung, the location of the primary infestation following the inhalation of fungal spores, into the tissue and can than affect all other organs of the body. Only modest possibilities are available for therapy of an invasive infection by A. fumigatus. Consequently, the disease leads to the death of the patient in more than 70 percent of the cases. But even if the infection spreads only on the surface of the body, the fungal infections are not only unpleasant but also dangerous in such location. Fungi take in glucose from the blood with their radices and segregate partially toxic metabolites, so called mycotoxins, damaging the liver. Thus, fungal infections have to be treated.

Fungal infections also represent a big problem in animals (e.g. farm animals, birds, fish, small animals and zoo animals as well as invertebrates like bees) where they can cause diseases that have to be treated.

Fungi, like their hosts, are eukaryotic organisms and thus, they are considerably more difficult to combat without compromising the human or animal organism as compared to bacteria. The treatment of Candida infections is particularly difficult as this microorganism is particularly flexible and permanently changes its appearance. In doing so it changes from a small spheroidal cell form, the yeast type, to a long multi cellular filaments, the hypha form. In particular this hypha form is particularly dangerous as it is able to penetrate tissue.

The treatment of fungal diseases often takes a long time. The duration of treatment depends on the type of fungus causing the infection. Therefore, therapy is accompanied by the identification of the fungus and the description of its characteristics, in particular its resistance against antifungal agents, in laboratory experiments.

Antifungal agents (antimycotica) can in principle have two different effects. They can lead to inhibition of fungal proliferation and thus act fungistatically. But they can also have fungicidal action and thus, exert a killing effect on the fungi. Most of the known antifungal agents interfere with the synthesis of the substance ergosterol, which is an important component of the fungal cell membrane. Ergosterol synthesis takes place in several steps, wherein different antifungal agents can interfere with different stages of synthesis. The interference with the ergosterol synthesis can lead to inhibition of fungal growth and can even lead to killing of the fungi.

Azole derivatives inhibit the biosynthesis of ergosterol, an important component of the fungal cell membrane, by interfering with the cytochrome P450 system. The effect of most azole derivatives is fungistatic, some also have fungicidal properties. Azole-derivatives are mainly used locally but are also suitable for systemic treatment. In this context one must pay attention that the active ingredients must not reach the bone tissue or the central nervous system.

Locally applied active ingredients are e.g. clotrimazole, bifonazole and econazole. All three substances are effective against yeasts, moulds and dermatophytes.

The systemic intake is oral or in form of injections or infusions. Indications are for example systemic mycosis, onychomycosis and severe mucocutanic Candida mycosis, in particular in case of AIDS and immune defects. For this purpose medicaments like fluconazole, ketoconazole and others are available. Amongst experts fluconazole these days represents the agent of first choice when Candida is detected in blood culture even if the species is not identified yet and the test for resistances is still missing.

Inhibitors of squalene epoxidase also interfere with ergosterol synthesis. Their effect is fungistatic. In case of dermatophytes, these substances are also fungicidal. Terbinafine and naftifine belong to these medicaments.

Medicaments belonging to the group of morpholine derivatives (amorolfine) are preferably used for the treatment of dermatophyte diseases.

The polyene derivatives form complexes (bonding) with sterols of the fungal membranes and thus disturb membrane functions. In case of the systemic administration of polyene derivatives it is problematic that the fungal sterols are very similar to sterols of the human cell membrane. Therefore, only such polyene derivatives can be used for a systemic treatment that have only a low binding capacity to the human scaffolds, like cholesterol, to avoid damaging these substances. The most widely used agents are amphotericin B and nystatin.

Amphotericin B is a broadband antimycotic agent whose effect and function is amply described in patent US 2,908,611. However, its use is limited by its high kidney, liver and myelotoxicity. Because of its poor solubility it can be applied in form of colloids or liposome formulations (US 4,663,167; EP 0 421 733).

The prophylactic and therapeutic treatment of Candida infections by administration of probiotic agents is also known. Such probiotics contain lactate producing bacteria, which bacteria, or their metabolites, respectively, are thought to be responsible for the curative effect (WO99/17788; Perdigon et al., 1990). In particular, an immune stimulatory effect is presumed, which is desirable for defence reactions against fungal diseases (De Simone et al., 1993). Such an antimycotic therapy is indicated after administration of antibiotics against bacterial infections, as Candida pathogens damage the mucosa and suppress the immune defence.

Further, so called non-classical antimycotics have different mechanisms of action. US 6,414,035 describes the use of polyols like mannitol, sorbitol, or xylitol for the treatment and prophylaxis of fungal diseases of the mucosa.

WO97/07802 describes the use of an inhibitor of chitin synthesis (nikkomycin Z) against Candida pathogens.

In a further patent the use of complexing substances is recommended to combat fungi (GB 2033220). Complexing substances deprived fungi of zinc ions, required for their metabolism.

JP 08 325107 A discloses optically active aliphatic alpha-hydroxy carboxylic acids and their use as antimicrobial compounds.

WO 01/68085 A relates to a method for stimulating the immune system in a mammal, which comprises administering a sufficient amount of amino acid isoleucine or active isomers or analogs in particular by stimulating defensin production.

EP 0366 632 A1 discloses compounds which are used as nutrients and next have an antimicrobial effect.

Carballeira et al. 2004 deal with the synthesis and in vitro antifungal activity of the marine fatty acid (±)-2-Methoxytetradecanoic Acid.

EP 0273202 A1 discloses methods and compositions for enhancing the therapeutic effect of topically administered drugs. For this purpose, hydroxymonocarboxylic acids or related substances can be used as additives.

WO 2006/099325 A discloses a pharmaceutical composition for the treatment of otitis media and externa (middle or outer ear), with an effective amount of a) an ether of a saturated (C7-C14) - or an unsaturated (C8-C22) fatty alcohol with a polyol, b) an ester of a saturated (C7-C14) - or an unsaturated (C8-C22) fatty alcohol with a (C2-C8) hydroxycarboxylic acid, and/ or c) an alkoxylated derivative of the preceding compounds which have a free hydroxyl group,
and combinations of these compounds. As an amplifier (enhancer) mono-and disaccharides are mentioned.

WO 03/088955 A discloses a pharmaceutical composition comprising an alpha-keto carboxylic acid and lactic acid or a salt of lactic acid for the treatment of acute renal failure, bowel obstruction, cytokine mediated inflammatory diseases and other diseases.

Hall et al. 1977 describe experiments on the suitability of α-hydroxy-thiol esters as anti-tumor agents and as glyoxalase I inhibitors.

US 5,633,285 A discloses pharmaceutical compositions for wound healing with cytoprotective effect. These include Pyruvate, mixtures of saturated and unsaturated fatty acids, and antioxidants.

JP 58 213716 A discloses compounds consisting of certain alpha, beta-unsaturated fatty acids that can be used as carcinostatic agents.

US 5,827,882 A discloses refers to a method for dermatological treatment with ethyl pyruvate. An antifungal effect was shown only in conjunction with griseofulvin.

New approaches of antimycotic therapy are based on activating the body's own defence. By activation of human dendritic cells by fungal proteins or nucleic acids a cellular immune response is meant to be caused. The method has the disadvantage of being species specific (Bozza et al., 2004) and not having a broad antimycotic reaction.

A general problem in using antimycotics is the development of resistances (Sanglard and Odds, 2002). Resistances are closely related to the number of AIDS cases, bone marrow transplantations and chemotherapies (Randhawa, 2000). Candida glabrata and C. krusei are often resistant against fluconazole (Marr, 2004). Occurrence of resistance is attributed to increased expression of ABC-transporters transporting the medicament fluconanzole back out of the fungal cell (Bennett et al., 2004).

Different tests are available for the determination of fungal resistance. Amongst others, the standardized disc test according to Kirby-Bauer (Qin et al., 2004) or the determination of the minimal inhibition concentration (MIC) by VITEK 2 (bioMerieux, Marcy l'Etoile, France) are used for this purpose. The disc test according to Kirby-Bauer is based on the impregnation of filter discs and measuring of the corona of inhibition after applying the filters to inoculated agar plates. For the MIC test dilution series of the test substance are added to the nutrition medium containing defined concentrations of the fungus to be tested. The lowest inhibitor concentration which inhibits growth of the microorganism is determined. The tests should be in accordance with the NCCLS-guidelines (NCCLS, 1997).

Some antimycotics show severe side effects, in particular in the context of long term or high dose intake (Schwarze et al., 1998). In particular the administration of amphotericin B can be accompanied by angioedema, allergies, shaking chill, want of appetite, nausea, vomiting, partially severe anaemia, kidney and liver dysfunction, anaphylactic shock, loss of hearing or tinnitus. The application of antimycotics together with cisplatin, pentamidine, aminoglycosides can cause severe kidney damage.

New options for therapy are therefore more than desirable, in particular as amongst pathogenic fungi antibiotic resistance is increasing. For example, amongst the Candida species an increasing number of C. glabrata and C. krusei strains is resistant primarily to fluconazole (13th European Congress of Clinical Microbiology and Infectious Diseases, Glasgow, June 2003).

### SUMMARY OF THE INVENTION

The problem underlying the invention thus resides in providing compositions, for use as antifungal agents.

Accordingly, the present invention provides a pharmaceutical composition for topic administration comprising at least two pharmaceutically active compounds wherein one pharmaceutically active compound is a substance according to general formula (I) 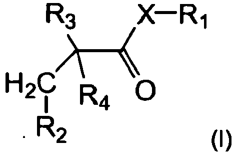
wherein X is O or S; and
R1 is a branched or non-branched alkyl, cycloalkyl, branched or non-branched alkenyl, cycloalkenyl, branched or non-branched alkinyl, cycloalkinyl, alkoxyalkyl, alkoxycarbonylalkyl, aryl or a sugar residue; and
R2 is H or a branched or non-branched alkyl, cycloalkyl, branched or non-branched alkenyl, cycloalkenyl, branched or non-branched alkinyl, cycloalkinyl, alkoxyalkyl, alkoxycarbonylalkyl or aryl residue; and
R3 and R4 together are =0,
or R3 is OH and R4 is H; or R3 is H and R4 is OH,
wherein said substance according to formula (I) is present in a therapeutically effective concentration and wherein the second pharmaceutically active compound is glucose
for use in the treatment and/or prophylaxis of fungal infections
with the proviso that when X=O, R2 is H and R3 or R4 is OH, R1 is not C1-C12 alkyl.

The anti-fungal effects encompass fungicidal and fungistatic action.

In one embodiment substances according to formula (I), wherein R1 comprises 1 to 8 carbon atoms and R2 is H or comprises 1 to 8 carbon atoms, or wherein R1 comprises 1 to 4 carbon atoms and R2 is H or comprises 1 to 2 carbon atoms are used.

In a further embodiment, the substance according to formula (I) is a substance wherein R1 and/or R2 is methyl, ethyl, propyl, isopropyl, butyl, or isobutyl. Particular embodiments according to the invention comprise one or more selected from methyl pyruvate, ethyl pyruvate, isopropyl pyruvate, butyl pyruvate, isobutyl pyruvate, ethyl 2- oxobutyrate, or the said pyruvate compound according to formula (I) wherein X = S, as well as compounds exemplified in table 1.

The invention also uses substances according to formula (I), wherein in said substance R3 or R4 is OH and it is selected from the group comprising the D-, L- enantiomer, and the racemic mixture thereof (equimolar as well as non-equimolar), as exemplified in table 2.

The invention relates to pharmaceutical compositions comprising one or more substances according to formula (I), and the use of said substances for the manufacture of a medicament.

In one embodiment, the pharmaceutical composition or medicament comprises one or more additional pharmaceutically active ingredients. Such ingredients can be selected from antifungal agents, such as one or more selected from fluconazole, ketoconazole, clotrimazole, bifonazole, econazole, itraconazole, terbinafine, naftifine, amorolfine, amphotericin B, nystatin, and nikkomycin, miconazolemiconazole, oxiconazole, sulconazole, flucytosine , griseofulvin, and biocidal peptides including defensines. The pharmaceutical composition or medicament can further comprise one or more auxiliary substances, including, but not limited to, fillers, flavouring agents and stabilizers. The pharmaceutical composition or medicament for use according the invention can be prepared in the form of galenic formulations commonly known in the art, including sustained release or controlled release galenic formulation.

The pharmaceutical composition or medicament is according to
the invention for topic administration, more particularly, for intraauricular, intranasal, percutaneous, transdermal, or pulmonary administration, or for administration as an aerosol, as mouth lavage, oil, cream, ointment, spray, gel and/or plaster,.

The pharmaceutical composition or medicament is for the use in the treatment and/or prophylaxis of fungal infections in animals, including invertebrates, non-mammalian vertebrates, mammalians and humans. The fungal infection can be resistant to antibiotic treatment, such as fluconazole, and may be an opportunistic infection. The fungal infection may be caused by one or more selected from the list comprising Candida spp., Aspergillus spp., Cryptococcus spp., Pneumocystis spp., Zygomyces spp., Dermatophytes, Blastomyces spp., Histoplasma spp., Coccidoides spp., Sporothrix spp.. Microsporidia spp, Malassezia spp and Basidiomycetes. Some of these infectious diseases may be an opportunistic infection, and/or may be characterized by antibiotic resistance.

In one embodiment, the infection is in an immunosuppressed animal, wherein said immunosuppression is associated with hereditary or acquired immune-defects, comprising acquired immune defect associated with HIV, organ transplantation, chemotherapy or exposure to radiation.

In one embodiment, the animal is concomitantly suffering from a bacterial or protozoal infection or worms, such as Trypanosoma, Leishmania, Plasmodium, Toxoplasma, helmithes, Acrobacter, Actinobacillus, Actinomyces, Bacteroides, Brucella, Clamydia, Clostridium, Campylobacter, Escherichia, Enterobacter, Enterococcus, Eubacterium, Fusobacterium, Helicobacter, Hemophilus, Legionella, Listeria, Mycobacteria, Mycoplasma, Neissaria, Pasteurella, Peptostreptococcus, Pneumococcus, Pneumocystis, Porphyromonas, Prevotella, Pseudomonas, Salmonella, Shigella, Spirochetes, Staphylococcus, Streptococcus, Treponema, Vibrio, Yersinia, Escherichia coli or Pneumocystis carinii. Some of these infectious diseases may be an opportunistic infection, and/or may be characterized by antibiotic resistance. In a further embodiment, the animal has a reduced blood glucose level.

According to the invention, the animal can be concomitantly suffering from cancer, and can be going to receive, is currently receiving, or has received conventional cancer therapy, comprising one or more of chemotherapy, surgery, radiotherapy or brachytherapy.

### DETAILED DESCRIPTION OF THE INVENTION

### A. Substances used in the invention

In the context of this application, the terms "substances" or "compounds" are used interchangeably.

The present invention relates to pharmaceutical composition for topic administration comprising at least two pharmaceutically active compounds
wherein one pharmaceutically active compound is a substance according to the general formula (I), 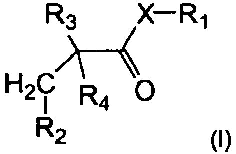
wherein X is O or S,
wherein R1 is a branched or non-branched alkyl, branched or non-branched alkenyl, branched or non-branched alkinyl, alkoxyalkyl, or alkoxycarbonylalkyl, each preferably with a chain length of C1 to C10, more preferably C1 to C8, more preferably C1 to C4, in particular C1, C2, C3 or C4;
or a cycloalkyl, cycloalkenyl, cycloalkinyl, aryl or a sugar residue, each preferably with a chain length of C3 to C10, more preferably C3 to C8, more preferably C3, C4, C5 or C6; and
R2 is H or a branched or non-branched alkyl, branched or non-branched alkenyl, branched or non-branched alkinyl, alkoxyalkyl, or alkoxycarbonylalkyl, each preferably with a chain length of C1 to C10, more preferably C1 to C8, more preferably C1 to C4, in particular C1, C2, C3 or C4;
or a cycloalkyl, cycloalkenyl, cycloalkinyl, or aryl residue, each preferably with a chain length of C3 to C10, more preferably C3 to C8, more preferably C3, C4, C5 or C6;; and
R3 and R4 together are =0,
or R3 is OH and R4 is H; or R3 is H and R4 is OH,
wherein said substance according to formula (I) is present in a therapeutically effective concentration and
and wherein the second pharmaceutically active compound is glucose
for use in the treatment and/or prophylaxis of fungal infections
with the proviso that when X=O, R2 is H and R3 or R4 is OH, R1 is not C1-C12 alkyl.

In one embodiment, R1 is not C1-C12 alkyl when X=O, R2 is H and R3 or R4 is OH.

In one embodiment the sugar in position R1 is substituted or non-substituted sugar.

In one embodiment R1 is not methyl, ethyl, propyl or butyl, when the treated subject is a fish, X is O, and R3 and R4 together are =0.

In one embodiment R1 comprises 1 to 4 carbon atoms and R2 is H or comprises 1 or 2 carbon atoms. In a further embodiment of the substance according to formula (I), R1 and/or R2 is methyl, ethyl, propyl, isopropyl, butyl, or isobutyl.

In one embodiment R2 is H, and R1 is methyl, ethyl, propyl, isopropyl, butyl, or isobutyl.

Specific examples of compounds used in the invention are listed in the following table 1, however, it is to be understood that this is not a limiting list. The skilled person can readily devise a large variety of additional compounds according to formula (I):

**Table 1: specific examples of compounds according to formula (I)**

| **R1** | **R2** | **R3 and R4** | **X** | **Name (I)** |
|---|---|---|---|---|
| **Alkyl** | | | | |
| Methyl | H | =O | =O | Methyl pyruvate |
| Ethyl | H | =O | =O | Ethyl pyruvate |
| Ethyl | H | =O | =S | S-Ethyl 2-oxopropanethionate |
| Propyl | H | =O | =S | S-Propyl 2-oxopropaneth ionate |
| Butyl | H | =O | =S | S-Butyl 2-oxopropanethionate |
| Propyl | H | =O | =O | Propyl pyruvate |
| Butyl | H | =O | =O | Butyl pyruvate |
| Pentyl | H | =O | =O | Pentyl pyruvate |
| Hexyl | H | =O | =O | Hexyl pyruvate |
| Heptyl | H | =O | =O | Heptyl pyruvate |
| Octyl | H | =O | =O | Octyl pyruvate |

| **Branched Alkyl** | | | | |
|---|---|---|---|---|
| Isopropyl | H | =O | =O | Isopropyl pyruvate |
| Isobutyl | H | =O | =O | Isobutyl pyruvate |
| Isopentyl | H | =O | =O | Isopentyl pyruvate |
| Isohexyl | H | =O | =O | Isohexyl pyrvate |
| Isoheptyl | H | =O | =O | Isoheptyl pyruvate |
| Isooctyl | H | =O | =O | Isooctyl pyruvate |

| **Cycloalkyl** | | | | |
|---|---|---|---|---|
| Cyclohexyl | H | =O | =O | Cyclohexyl pyruvate |
| Cyclohexyl methyl | H | =O | =O | Cyclohexylmethyl pyruvate |
| Cyclopentyl | H | =O | =O | Cyclopentyl pyrvate |
| Cyclopentyl methyl | H | =O | =O | Cyclopentylmethyl pyruvate |

| **Alkenyl residues** | | | | |
|---|---|---|---|---|
| Vinyl | H | =O | =O | Ethenyl pyruvate |
| Allyl | H | =O | =O | Propenyl pyruvate |
| Butenyl | H | =O | =O | Butenyl pyruvate |
| Pentenyl | H | =O | =O | Pentenyl pyruvate |
| Hexenyl | H | =O | =O | Hexenyl pyruvate |
| Heptenyl | H | =O | =O | Heptenyl pyruvate |
| Octenyl | H | =O | =O | Octenyl pyruvate |

| **Branched Alkenyl** | | | | |
|---|---|---|---|---|
| Isopropenyl | H | =O | =O | Isopropenyl pyruvate |
| Isobutenyl | H | =O | =O | Isobutenyl pyruvate |
| Isopentenyl | H | =O | =O | Isopentenyl pyruvate |
| Isohexenyl | H | =O | =O | Isohexenyl pyruvate |
| Isoheptenyl | H | =O | =O | Isoheptenyl pyruvate |
| IsooctenyL | H | =O | =O | Isooctenyl pyruvate |

| **Cycloalkenyl** | | | | |
|---|---|---|---|---|
| Cyclohexenyl | H | =O | =O | Cyclohexenyl pyruvate |
| Cyclohexenylmet hyl | H | =O | =O | Cyclohexenylmethyl pyruvate |
| Cyclo pentenyl | H | =O | =O | Cyclopentenyl pyruvate |
| Cyclopentenylme thyl | H | =O | =O | Cyclopentenylmethyl pyruvate |

| **Alkinyl** | | | | |
|---|---|---|---|---|
| Ethinyl | H | =O | =O | Ethinyl pyruvate |
| Propinyl | H | =O | =O | Propinyl pyruvate |
| Butinyl | H | =O | =O | Butinyl pyruvate |
| Pentinyl | H | =O | =O | Pentinyl pyruvate |
| Hexinyl | H | =O | =O | Hexinyl pyruvate |
| Heptinyl | H | =O | =O | Heptinyl pyruvate |
| Octinyl | H | =O | =O | Octinyl pyruvate |

| **Branched Alkinyl** | | | | |
|---|---|---|---|---|
| Isopentinyl | H | =O | =O | Isopentinyl pyruvate |
| Isohexinyl | H | =O | =O | Isohexinyl pyruvate |
| Isoheptinyl | H | =O | =O | Isoheptinyl pyruvate |
| Isooctinyl | H | =O | =O | Isooctinyl pyruvate |

| **Cycloalkinyl** | | | | |
|---|---|---|---|---|
| Cyclooctinyl | H | =O | =O | Cyclooctinyl pyruvate |
| **Alkoxyalkyl** | | | | |
| Methoxymethyl | H | =O | =O | Methoxymethyl pyruvate |
| Ethoxymethyl | H | =O | =O | Ethoxymethyl pyruvate |
| Methoxyethyl | H | =O | =O | Methoxyethyl pyruvate |

| **Alkoxycarbonyl alkyl** | | | | |
|---|---|---|---|---|
| Methoxycarbonyl methyl | H | =O | =O | Methoxycarbonylmethyl pyruvate |
| Ethoxycarbonylm ethyl | H | =O | =O | Ethoxycarbonylmethyl pyruvate |

| **Aryl** | | | | |
|---|---|---|---|---|
| Phenyl | H | =O | =O | Phenyl pyruvate |
| Naphthyl | H | =O | =O | Naphthyl pyruvate |
| **Sugar** | H | | | |
| Glucosyl | H | =O | =O | Gucosyl pyruvate |
| Galactosyl | H | =O | =O | Galactosyl pyruvate |
| Mannosyl | H | =O | =O | Mannosyl pyruvate |

| **Alkyl** | **Alkyl** | | | |
|---|---|---|---|---|
| Methyl | Methyl | =O | =O | Methyl 2-oxobutanoat |
| Ethyl | Methyl | =O | =O | Ethyl 2-oxobutanoat |
| Ethyl | Ethyl | =O | =S | S-Ethyl 2-oxopentanethionate |
| Propyl | Methyl | =O | =O | Propyl2-oxobutanoat |
| Butyl | Methyl | =O | =O | Butyl 2-oxobutanoat |
| Methyl | Ethyl | =O | =O | Methyl 2-oxopentanoate |
| Ethyl | Ethyl | =O | =O | Ethyl 2-oxopentanoate |
| Propyl | Ethyl | =O | =O | Propyl 2-oxopentanoate |
| Butyl | Ethyl | =O | =O | Butyl 2-oxopentanoate |
| Methyl | Propyl | =O | =O | Methyl 2-oxohexanoate |
| Ethyl | Propyl | =O | =O | Ethyl 2-oxohexanoate |
| Propyl | Propyl | =O | =O | Propyl 2-oxohexanoate |
| Butyl | Propyl | =O | =O | Butyl 2-oxohexanoate |
| Methyl | Butyl | =O | =O | Methyl 2-oxoheptanoate |
| Ethyl | Butyl | =O | =O | Ethyl 2-oxoheptanoate |
| Propyl | Butyl | =O | =O | Propyl 2-oxoheptanoate |
| Butyl | Butyl | =O | =O | Butyl 2-oxoheptanoate |

| **Branched Alkyl** | **Alkyl** | | | |
|---|---|---|---|---|
| Isobutyl | Methyl | =O | =O | Isobutyl 2-oxobutanoate |
| Isobutyl | Ethyl | =O | =O | Isobutyl 2-oxopentanoate |
| Isobutyl | Propyl | =O | =O | Isobutyl 2-oxohexanoate |
| Isobutyl | Butyl | =O | =O | Isobutyl 2-oxoheptanoate |
| **Cyclo Alkyl** | **Alkyl** | | | |
| Cyclohexyl | Ethyl | =O | =O | Cyclohexyl 2-oxopentanoate |
| Cyclohexyl methyl | Ethyl | =O | =O | Cyclohexylmethyl 2-oxopentanoate |

| **Sugar** | **Alkyl** | | | |
|---|---|---|---|---|
| Glucosyl | Methyl | =O | =O | Glucosyl 2-oxobutanoate |
| Glucosyl | Ethyl | =O | =O | Glucosyl 2-oxopentanoate |

| **Alkyl** | **Alkenyl** | | | |
|---|---|---|---|---|
| Propyl | Butenyl | =O | =O | Propyl 2-oxoheptenoate |
| **Cycloalkyl** | **Alkenyl** | | | |
| Cyclohexyl | Butenyl | =O | =O | Cyclohexyl 2-oxoheptenoate |
| **Alkyl** | **Alkinyl** | | | |
| Propyl | Butinyl | =O | =O | Propyl 2-oxoheptinoate |
| Butyl | Butinyl | =O | =O | Butyl 2-oxoheptinoate |
| **Cycloalkyl** | **Alkinyl** | | | |
| Cyclohexyl | Butinyl | =O | =O | Cyclohexyl 2-oxoheptinoate |

| **Alkoxyalkyl** | **Alkyl** | | | |
|---|---|---|---|---|
| Methoxymethyl | Ethyl | =O | =O | Methoxymethyl 2-oxopentanoate |
| Ethoxymethyl | Ethyl | =O | =O | Ethoxymethyl 2-oxopentanoate |
| Methoxyethyl | Ethyl | =O | =O | Methoxyethyl 2-oxopentanoate |

| **Alkoxycarbonyl alkyl** | **Alkyl** | | | |
|---|---|---|---|---|
| Methoxycarbonyl methyl | Ethyl | =O | =O | Methoxycarbonylmethyl 2-oxopentanoate |
| Ethoxycarbonylm ethyl | Ethyl | =O | =O | Ethoxycarbonylmethyl 2-oxopentanoate |

| **Alkyl** | **Alkoxycarbonyl alkyl** | | | |
|---|---|---|---|---|
| Ethyl | Methoxycarbonyl methyl | =O | =O | Ethyl-4-methoxycarbonyl-2-oxobutanoate |
| Ethyl | Ethoxycarbonylm ethyl | =O | =O | Ethyl-4-ethoxycarbonyl-2-oxobutanoate |

| **Alkyl** | **Alkoxyalkyl** | | | |
|---|---|---|---|---|
| Ethyl | Methoxy methyl | =O | =O | Ethyl 4-methoxy-2-oxobutanoate |
| Ethyl | Ethoxymethyl | =O | =O | Ethyl4-ethoxy-2-oxobutanoate |
| Ethyl | Methoxyethyl | =O | =O | Ethyl 5-methoxy-2-oxopentanoate |

Particular examples of substances according to formula (I) comprise methyl pyruvate, ethyl pyruvate, propyl pyruvate, butyl pyruvate, pentyl pyruvate, hexyl pyruvate, octyl pyruvate, isobutyl pyruvate,isopentyl pyruvate, isohexyl pyruvate, isoheptyl pyrvate, isooctyl pyruvate, cyclopentyl pyruvate, cyclopentylmethyl pyruvate, cyclohexyl pyruvate, cyclohexylmethyl pyruvate, butenyl pyruvate, hexenyl pyruvate, isobutenyl pyruvate, isohexenyl pyruvate, butinyl pyruvate, hexinyl pyruvate, methoxymethyl pyruvate, ethoxymethyl pyruvate, ethoxycarbonylmethyl pyruvate, methyl-2-oxobutanoate, ethyl 2-oxobutanoate, butyl-2-oxo-butanoate, methyl-2-oxopentanoate, ethyl-2-oxoheptanoate, butyl-2-oxopentanoate, methyl-2-oxohexanoate, ethyl-2-oxohexanoate,butyl-2-oxohexanoate, methyl-2-oxoheptanoate, ethyl-2-oxoheptanoate, butyl-2-oxo-heptanoate, isobutyl-2-oxobutanoate, isobutyl-2-oxohexanoate, cyclohexyl-2-oxopentanoate, cyclohexylmethyl-2-oxopentanoate, propyl-2-oxoheptenoate, cyclohexyl-2-oxoheptenoate, butyl-2-oxoheptinoate, methoxymethyl-2-oxopantanoate, ethoxycarbonylmethyl-2-oxopentanoate,ethyl-4-methoxycarbonyl-2-oxobutanoate, ethyl-4-methoxy-2-oxobutanoate, or the said compounds wherein X = S, and/or the said compound wherein R3 or R4 is OH.

Preferred examples of substances used in the invention comprise methyl pyruvate, ethyl pyruvate, propyl pyruvate, butyl pyruvate, pentyl pyruvate, hexyl pyruvate,isopropyl pyruvate, isobutyl pyruvate, isopentyl pyruvate, isohexyl pyruvate, methyl-2-oxobutanoate, methyl-2-oxopentanoate, ethyl-2-oxobutanoate, butyl-2-oxobutanoate, ethyl-2-oxopentanoate. cyclohexylmethyl pyruvate,
or the said compounds wherein X = S, and/or the said compound wherein R3 or R4 is OH.

More preferred compounds used in the invention comprise methyl pyruvate, ethyl pyruvate, propyl pyruvate, butyl pyruvate, isobutyl pyruvate, ethyl-2-oxobutanoate, ethyl-2-oxopantanoate, cyclohexylmethyl pyruvate,
or the said compounds wherein X = S, and/or the said compound wherein R3 or R4 is OH.

Particularly preferred compounds are methyl pyruvate, ethyl pyruvate, butyl pyruvate, isobutyl pyruvate and ethyl-2-oxo-butyrate
or the said compounds wherein X = S, in particular S-ethyl pyruvate, and/or the said compounds wherein R3 or R4 together are -OH.

### a) Substances according to formula (I) inhibit glyoxalases

Surprisingly it was found that the substances according to formula (I) inhibit glyoxalase I and/or II.

Fungal cells generate energy by the degradation of different food stuffs, and store it as chemical energy in energy rich compounds, particularly in the form of ATP. These energy rich compounds are subject to extensive turnover interconnected with anabolic and catabolic processes, by being used, for example, in the synthesis of proteins, nucleic acids, sugars, lipids etc., the transport of substances against concentration gradients and regulatory activities, and are formed anew in certain metabolic pathways. A plurality of compounds can serve as energy providing substances, the most important being sugars and fatty acids. After metabolising the different monosaccharides and their di-, oligo- and polymers extra- or intracellularly into corresponding derivatives, sugar degradation takes place in glycolysis. Glycolysis allows anaerobic as well as, in combination with oxidative phosphorylation, aerobic energy generation.

Glycolysis, however, is always accompanied by the formation of oxoaldehydes, in particular of methylglyoxal. These compounds are highly toxic as they easily form adducts with cellular proteins and nucleic acids and lead to their inactivation. Therefore, all cells using glycolysis employ detoxification systems, in most cases consisting of the enzymes glyoxalase I and II.

Both glyoxalases I and II are responsible for the degradation of the side product of glycolysis, methylglyoxal. Methylglyoxal is cytotoxic (e.g. by the formation of adducts with cellular proteins and nucleic acids). Inhibition of the degradation of methylglyoxal leads to inhibition of cell proliferation and cell death by different mechanisms.

Thus, in one embodiment the substances according to formula (I) are for the inhibition of glyoxalase I and / or II, advantageously I and II. The inhibition of multiple enzymes drastically reduces the probability of developing resistance within the therapeutic period.

The compounds according formula (I) inhibit glyoxalase I and / or II, advantageously I and II. The inhibition of multiple enzymes drastically reduces the probability of developing resistance within the therapeutic period.

Surprisingly it was found that compounds according formula (I) like e.g. ethyl pyruvate are capable of inhibiting glyoxalase I as well as glyoxalase II. Inhibition of glyoxalases by compounds of the present invention inhibits the cellular detoxification of methylglyoxal and via various mechanisms leads to the inhibition of cell proliferation and to cell death.

Advantageously, compounds according formula (I) inhibit such cells showing a clearly increased rate of glycolysis whereas the metabolism of cells with a normal rate of glycolysis is not or only slightly affected.

Glyoxalase I (GL01, alternatively abbreviated as Gly I,) is also known as (R)-S-lactoylglytythione methyl-glyoxal-lyase EC4.4.1.5), glyoxalase II (GL02, alternatively abbreviated as Gly II) is also known as S-2-hydroxy-acylglutathione hydrolase (EC 3.1.2.6).

Glyoxalases are phylogenetically highly conserved at the amino acid and genetic level. As used herein, the term "glyoxalase" refers to the mammalian enzymes glyoxalase I and/or II, as well as to the respective glyoxalases of non-mammalian eukaryotic and prokaryotic organisms, such as glyoxalase I and II of fungi like yeast or other microorganisms.

Thus, the term "inhibiting glyoxalase I and/or II" encompasses the inhibition of the mammalian as well as the respective non-mammalian enzymes.

According to the invention, the substances according to formula (I) are for direct inhibition of glyoxalase I and/or II when R3 and R4 together are =0.

In contrast, when R3 or R4 are -OH, said substances according to formula (I) do not directly inhibit glyoxalase I and/or II. Rather, said substances, also called "prodrugs", are transformed, i.e. oxidized, to a substance wherein R3 and R4 together are =0.

Said transformation/oxidization can be effected ex vivo, e.g. by means of a chemical oxidant, such as potassium permanganate. Other suitable oxidants are for example hydrogen peroxide, iodine, iodide benzoic acid and others.

Alternatively, said transformation takes place in the organism, or on the skin or mucosa of the mammal upon administration of said compound. Such transformation is effected e.g. via dehydrogenases, in particular via lactate dehydrogenase (Lluis and Bozal, 1976).

Compounds of formula (I), wherein R3 or R4 is -OH, and which undergo transformation such that R3 and R4 together are =0 are for example compounds of the general formula (II) and/or (III), 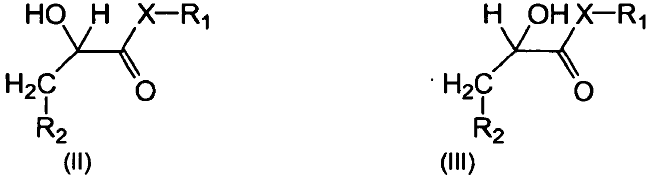 wherein X, R1 and R2 are defined as in formula (I), above.

Specific compounds of the general formula (II) and/or (III) are for example methyl lactate, propyl lactate, butyl lactate, ethyl lactate, and ethyl-2-hydroxybutanoate, which are transformed into, e.g. butyl pyruvate, ethyl pyruvate, and ethyl-2-oxobutanoate, respectively,

When in a substance according to formula (I) R3 or R4 is OH, the invention encompasses the D-, L- enantiomer and the racemic mixture thereof. In the context of this invention, equimolar as well as non-equimolar mixtures of corresponding enantiomers are to be considered as racemic mixtures.

In other words, in case the compounds according formula (I) are compounds with one or more chiral centres, for example ethyl lactate or butyl lactate, the corresponding D- and L-isomers can be used as well as racemic mixtures, for example ethyl D-lactate (DEL), ethyl L-lactate (LEL) or racemic mixtures of DEL and LEL, and butyl D-lactate (DBL), butyl L-lactate (LBL) or racemic mixtures of DBL and LBL, respectively.

Specific examples of compounds according formula (I) are listed in the following table 2, however, it is to be understood that this is not a limiting list. The skilled person can readily devise a large variety of additional compounds according to formula (II/III). The following table is understood to encompass the D- or L- Isomers or racemic mixtures of the listed substances. In other words, substances according to either formula II or formula III are specifically disclosed:

**Table 2: specific compounds according to formula II / III:**

| **R1** | **R2** | **R3/R4** | **X** | **Name** |
|---|---|---|---|---|
| **Alkyl** | | | | |
| Methyl | H | H/OH | =O | Methyl lactate |
| Ethyl | H | H/OH | =O | Ethyl lactate |
| Propyl | H | H/OH | =O | Propyl lactate |
| Butyl | H | H/OH | =O | Butyl lactate |
| Pentyl | H | H/OH | =O | Pentyl lactate |
| Hexyl | H | H/OH | =O | Hexyl lactate |
| Heptyl | H | H/OH | =O | Heptyl lactate |
| Octyl | H | H/OH | =O | Octyl lactate |
| Ethyl | H | H/OH | =S | S-Ethyl 2-hydroxypropanethionate |
| Propyl | H | H/OH | =S | S-propyl 2-hydroxypropanethionate |
| Butyl | H | H/OH | =S | S-Butyl 2-hydroxypropanethionate |

| **Branched Alkyl** | | | | |
|---|---|---|---|---|
| Isopropyl | H | H/OH | =O | Isopropyl lactate |
| Isobutyl | H | H/OH | =O | Isobutyl lactate |
| Isopentyl | H | H/OH | =O | Isopentyl lactate |
| Isohexyl | H | H/OH | =O | Isohexyl lactate |
| Isoheptyl | H | H/OH | =O | Isoheptyl lactate |
| Isooctyl | H | H/OH | =O | Isooctyl lactate |

| **Cycloalkyl** | | | | |
|---|---|---|---|---|
| Cyclohexyl | H | H/OH | =O | Cyclohexyl lactate |
| Cyclohexylmethyl | H | H/OH | =O | Cyclohexylmethyl lactate |
| Cyclopentyl | H | H/OH | =O | Cyclopentyl lactate |
| Cyclopentyl methyl | H | H/OH | =O | Cyclopentylmethyl lactate |
| Cyclopropyl | H | H/OH | =O | Cyclopropyl lactate |
| Cyclopropyl methyl | H | H/OH | =O | Cyclopropylmethyl lactate |

| **Alkenyl** | | | | |
|---|---|---|---|---|
| Vinyl | H | H/OH | =O | Vinyl lactate |
| Allyl | H | H/OH | =O | Propenyl lactate |
| Butenyl | H | H/OH | =O | Butenyl lactate |
| Pentenyl | H | H/OH | =O | Pentenyl lactate |
| Hexenyl | H | H/OH | =O | Hexenyl lactate |
| Heptenyl | H | H/OH | =O | Heptenyl lactate |
| Octenyl | H | H/OH | =O | Octenyl lactate |

| **Branched Alkenyl** | | | | |
|---|---|---|---|---|
| Isopropenyl | H | H/OH | =O | Isopropenyl lactate |
| Isobutenyl | H | H/OH | =O | Isobutenyl lactate |
| Isopentenyl | H | H/OH | =O | Isopentenyl lactate |
| Isohexenyl | H | H/OH | =O | Isohexenyl lactate |
| Isoheptenyl | H | H/OH | =O | Isoheptenyl lactate |
| IsooctenyL | H | H/OH | =O | Isooctenyl lactate |
| **Cycloalkenyl** | | | | |
| Cyclohexenyl | H | H/OH | =O | Cyclohexenyl lactate |
| Cyclohexenylmethyl | H | H/OH | =O | Cyclohexenylmethyl lactate |
| Cyclopentenyl | H | H/OH | =O | Cyclopentenyl lactate |
| Cyclopentenylmethyl | H | H/OH | =O | Cyclopentenylmethyl lactate |

| **Alkinyl** | | | | |
|---|---|---|---|---|
| Ethinyl | H | H/OH | =O | Ethinyl lactate |
| Propinyl | H | H/OH | =O | Propinyl lactate |
| Butinyl | H | H/OH | =O | Butinyl lactate |
| Pentinyl | H | H/OH | =O | Pentinyl lactate |
| Hexinyl | H | H/OH | =O | Hexinyl lactate |
| Heptinyl | H | H/OH | =O | Heptinyl lactate |
| Octinyl | OH | H/OH | =O | Octinyl lactate |

| **Branched Alkinyl** | | | | |
|---|---|---|---|---|
| Isopentinyl | H | H/OH | =O | Isopentinyl lactate |
| Isohexinyl | H | H/OH | =O | Isohexinyl lactate |
| Isoheptinyl | H | H/OH | =O | Isoheptinyl lactate |
| Isooctinyl | H | H/OH | =O | Isooctinyl lactate |

| **Cycloalkinyl** | | | | |
|---|---|---|---|---|
| Cyclooctinyl | H | H/OH | =O | Cyclooctinyl lactate |

| **Alkoxyalkyl** | | | | |
|---|---|---|---|---|
| Methoxymethyl | H | H / OH | =O | Methoxymethyl lactate |
| Ethoxymethyl | H | H / OH | =O | Ethoxymethyl lactate |
| Methoxyethyl | H | H / OH | =O | Methoxyethyl lactate |

| **Alkoxycarbonylalkyl** | | | | |
|---|---|---|---|---|
| Methoxycarbonylmeth yl | H | H / OH | =O | Methoxycarbonylmethyl lactate |
| Ethoxycarbonylmethyl | H | H / OH | =O | Ethoxycarbonylmethyl lactate |

| **Aryl** | | | | |
|---|---|---|---|---|
| Phenyl | H | H / OH | =O | Phenyl lactate |
| Naphthyl | H | H / OH | =O | Naphthyl lactate |
| **Sugar** | | | | |
| Glucosyl | H | H / OH | =O | Gucosyl lactate |
| Galactosyl | H | H / OH | =O | Galactosyl lactate |
| Mannosyl | **H** | H / OH | =O | Mannosyl lactate |

| **Alkyl** | **Alkyl** | | | |
|---|---|---|---|---|
| Methyl | Methyl | H / OH | =O | Methyl 2-hydroxybutanoate |
| Ethyl | Methyl | H / OH | =O | Ethyl 2-hydroxybutanoate |
| Propyl | Methyl | H / OH | =O | Propyl 2-hydroxybutanoate |
| Butyl | Methyl | H / OH | =O | Butyl 2-hydroxybutanoate |
| Methyl | Ethyl | H / OH | =O | Methyl 2-hydroxypentanoate |
| Ethyl | Ethyl | H / OH | =O | Ethyl 2-hydroxypentanoate |
| Ethyl | Ethyl | H / OH | =S | S-Ethyl 2-hydrxypentanethionate |
| Propyl | Ethyl | H / OH | =O | Propyl 2-hydroxypentanoate |
| Butyl | Ethyl | H / OH | =O | Butyl 2-hydroxypentanoate |
| Methyl | Propyl | H / OH | =O | Methyl 2-hydroxyhexanoate |
| Ethyl | Propyl | H / OH | =O | Ethyl 2-hydroxyhexanoate |
| Propyl | Propyl | H / OH | =O | Propyl 2-hydroxyhexanoate |
| Butyl | Propyl | H / OH | =O | Butyl 2-hydroxyhexanoate |
| Methyl | Butyl | H / OH | =O | Methyl 2-hydroxyheptanoate |
| Ethyl | Butyl | H / OH | =O | Ethyl 2-hydroxyheptanoate |
| Propyl | Butyl | H / OH | =O | Propyl 2-hydroxyheptanoate |
| Butyl | Butyl | H / OH | =O | Butyl 2-hydroxyheptanoate |

| **Branched Alkyl** | | | | |
|---|---|---|---|---|
| Isobutyl | Methyl | H / OH | =O | Isobutyl 2-hydroxybutanoate |
| Isobutyl | Ethyl | H / OH | =O | Isobutyl 2-hydroxypentanoate |
| Isobutyl | Propyl | H / OH | =O | Isobutyl 2-hydroxyhexanoate |
| Isobutyl | Butyl | H / OH | =O | Isobutyl 2-hydroxyheptanoate |

| **Cycloalkyl** | | | | |
|---|---|---|---|---|
| Cyclohexyl | Ethyl | H / OH | =O | Cyclohexyl 2-hydroxypentanoate |
| Cyclohexylmethyl | Ethyl | H / OH | =O | Cyclohexylmethyl 2-hydroxypentanoate |

| **Sugar** | **Alkyl** | | | |
|---|---|---|---|---|
| Glucosyl | Methyl | H / OH | =O | Glucosyl 2-hydroxybutanoate |
| Glucosyl | Ethyl | H / OH | =O | Glucosyl 2-hydroxypentanoate |

| **Alkyl** | **Alkenyl** | | | |
|---|---|---|---|---|
| Propyl | Butenyl | H / OH | =O | Propyl 2-hydroxyheptenoate |

| **Cycloalkyl** | **Alkenyl** | | | |
|---|---|---|---|---|
| Cyclohexyl | Butenyl | H / OH | =O | Cyclohexyl 2-hydroxaheptenoate |

| **Alkyl** | **Alkinyl** | | | |
|---|---|---|---|---|
| Propyl | Butinyl | H / OH | =O | Propyl 2-hydroxyheptinoate |
| Butyl | Butinyl | H / OH | =O | Butyl 2-hydroxyheptinoate |

| **Cycloalkyl** | **Alkinyl** | | | |
|---|---|---|---|---|
| Cyclohexyl | Butinyl | H / OH | =O | Cyclohexyl 2-hydroxyheptinoate |

| **Alkoxyalkyl** | **Alkyl** | | | |
|---|---|---|---|---|
| Methoxymethyl | Ethyl | H / OH | =O | Methoxymethyl 2-hydroxypentanoate |
| Ethoxymethyl | Ethyl | H / OH | =O | Ethoxymethyl 2-hydroxypentanoate |
| Methoxyethyl | Ethyl | H / OH | =O | Methoxyethyl 2-hydroxypentanoate |

| **Alkoxycarbonylalkyl** | **Alkyl** | | | |
|---|---|---|---|---|
| Methoxycarbonylmeth yl | Ethyl | H / OH | =O | Methoxycarbonylmethyl 2-hydroxypentanoate |
| Ethoxycarbonylmethyl | Ethyl | H / OH | =O | Ethoxycarbonylmethyl 2-hydroxypentanoate |

| **Alkyl** | **Alkoxycar bonylalkyl** | | | |
|---|---|---|---|---|
| Ethyl | Methoxycar bonylmethy l | H / OH | =O | Ethyl-4-methoxycarbonyl-2-hydroxybutanoate |
| Ethyl | Ethoxycarb onylmethyl | H / OH | =O | Ethyl-4-ethoxycarbonyl-2-hydroxybutanoate |

| **Alkyl** | **Alkoxyalky l** | | | |
|---|---|---|---|---|
| Ethyl | Methoxyme thyl | H / OH | =O | Ethyl 4-methoxy-2-hydroxybutanoate |
| Ethyl | Ethoxymeth yl | H / OH | =O | Ethyl 4-ethoxy-2-hydroxybutanoate |
| Ethyl | Methoxyeth yl | H / OH | =O | Ethyl 5-methoxy 2-hydroxypentanoate |

The D- or L- enantiomers or the racemic mixtures thereof of the following substances are further particular examples of substances used in the invention: methyl lactate, ethyl lactate, propyl lactate, butyl lactate, pentyl lactate, hexyl lactate, octyl lactate, isobutyl lactate, isopentyl lactate, isohexyl lactate, isoheptyl lactate, isooctyl lactate, cyclopentyl lactate, cyclopentylmethyl lactate, cyclohexyl lactate, cyclohexylmethyl lactate, butenyl lactate, hexenyl lactate, isobutenyl lactate,isohexenyl lactate, butinyl lactate, hexinyl lactate, methoxymethyl lactate, ethoxymethyl lactate, ethoxycarbonylmethyl lactate, methyl-2-hydroxybutanoate, ethyl 2-hydroxybutanoate, butyl-2-hydroxybutanoate, methyl-2-hydroxypentanoate, ethyl-2-hydroxyheptanoate, butyl-2-hydroxypentanoate, methyl-2-hydroxyhexanoate, ethyl-2-hydroxyhexanoate,butyl-2-hydroxyhexanoate, methyl-2-hydroxyheptanoate, ethyl-2-hydroxyheptanoate, butyl-2-hydroxyheptanoate,isobutyl-2-hydroxybutanoate, isobutyl-2-hydroxyhexanoate, cyclohexyl-2-hydroxypentanoate, cyclohexylmethyl-2-hydroxypentanoate, propyl-2-hydroxyheptenoate, cyclohexyl-2-hydroxyheptenoate, butyl-2-hydroxyheptinoate, methoxymethyl-2-hydroxypantanoate, ethoxycarbonylmethyl-2-hydroxypentanoate,ethyl-4-methoxycarbonyl-2-hydroxybutanoate, ethyl-4-methoxy-2-hydroxybutanoate, or the said compounds, wherein X=S.

If ethyl lactate is used, ethyl L-lactate (LEL) as well as ethyl D-lactate (DEL) are effective. The effect of esters of D-lactate is surprising as D-lactate is considered to be non-metabolizable in mammalian cells (Murray et al., 1993) and the same had to also be presumed for esters of D-lactate. Hence it could not have been expected that those compounds could be applied at all according to the invention and that they would exhibit such good effectiveness.

The inventors' own measurements confirm the interconversion of ethyl lactate and ethyl pyruvate by NAD-dependent lactate dehydrogenases. Butyl lactate can, to a lesser degree than ethyl lactate, also be transformed by NAD-dependent lactate dehydrogenases. When butyl lactate is used according to the invention only cells with a particularly high activity of lactate dehydrogenase will reach therapeutically effective concentrations of butyl pyruvate.

Thus, compounds of the general formula (II) and (III) act as prodrugs, as exemplified in Examples 2 and 3.

Lactate and alkyl lactate, respectively, are transported over the cell membrane by a lactate shuttle (monocarboxylate transporters (MCT's)) (Garcia et al., 1994; von Grumbckow et al., 1999) in combination with a proton transporter. For the transport into mitochondria mitochondrial MCTs are available. Addition of lactate and its alkyl esters, respectively, to blood leads to slight alkalization due to the proton-connected lactate transporters whereas the application of pyruvate and its alkyl esters, respectively, leads to an acidosis of blood, caused by enzymatic ester cleavage. Lactate and alkyl lactate are transported stereo selectively and better through the membrane as compared to pyruvate and alkyl pyruvate (Roth and Brooks, 1990). Alkyl pyruvates administered to blood have to be transformed into alkyl lactates before they can enter cells.

Moreover, the rate of hydrolysis of compounds according to formula (I) wherein R3 or R4 is - OH is lower as compared to compounds wherein R3 and R4 together are =0, leading to an improved in vivo stability.

Therefore, it is advantageous to use compounds wherein R3 or R4 is -OH, and in particular, therapeutically active, physiologically compatible alkyl lactates.

In the context of this application, the compounds according to general formula (I), including direct glyoxalase inhibitors and their prodrugs, and the specific examples of compounds, including the compounds according to formula (II) and (III), are also summarily referred to as "compounds used in the invention".

A particular advantage of the substances used in the invention resides in the fact that toxicity of said substances and their metabolites is only very low (Clary et al., 1998). After saponification by esterases they are metabolized to equally non- or only slightly toxic alcohols and to carboxylic acids which are also produced in normal cell metabolism (e.g. pyruvate and lactate). For example, the concentration of lactate in human blood is 2-20 mM. Lactate is contained in many foods, is generated in metabolism and can be metabolised.

This also explains the low or even absent ecotoxicity of these compounds (Bowmer et al., 1998) in tests with Selenastrum capricomutum, Daphnia magna, Pimephales promelas and Brachydanio rerio. These compounds are also devoid of mutagenic potential in normal cells as demonstrated in an established test system (Andersen and Jensen, 1984).

### b) The substances of formula (I) differ from known inhibitors of glyoxalases

The inhibition of glyoxalases by compounds according to formula (I) has so far been unknown.

On the basis of the substrate of glyoxalase I, the hemithioacetal of methylglyoxal and glutathione, peptidic glyoxalase inhibitors are widely described in the literature (Creighton et al, 2003; Hamilton & Creighton, 1992; Hamilton and Batist, 2004; Johansson et al., 2000; Kalsi et al., 2000; Kamiya et al, 2005; Ranganathan et al, 1995; Sharkey et al., 2000; Thornalley, 1993; Thornalley et al, 1996; Thornalley, 1996; Vince and Daluge, 1970).

US 4,898,870 describes pyrroloquinoline quinone compounds in the context of inhibition of glyoxalase I. WO 99/035128 also describes compounds for inhibition of glyoxalase I. WO 04/101506 describes a further class of non-peptidic inhibitors of glyoxalase I, as does Douglas et al (1985).

However, the glyoxalase inhibitors known so far exhibit a relatively high or very high toxicity and are metabolized to compounds which in turn have manifold pharmacological effects, some of which lead to severe side effects.

Furthermore, the glyoxalase inhibitors known so far only inhibit either glyoxalase I or glyoxalase II, respectively. However, when inhibitors are directed to a single protein target only, resistance can develop very quickly, as for example mutations appear in the relevant protein, which make the inhibitor ineffective.

Therefore, the glyoxalase inhibitors used in the present invention are advantageous over known inhibitors.

### c) The known effects of substances according to formula (I) do not encompass glyoxalase inhibition

From the known effect of methyl pyruvate its influence on glyoxalases was not predictable. For years methyl pyruvate has been intensely investigated as an insulinotropic compound (Düfer et al., 2002; Valverde et al., 2001; Lembert et al., 2001). This effect is mediated by influencing potassium channels and mitochondrial effects. Inhibitory effects on LDH have also been proposed (Lluis and Bozal, 1976).

Furthermore, it has been described that the administration of ethyl pyruvate can improve inflammatory states, reperfusion injury, acute renal failure and ischemia (WO 03/088955; WO 02/074301; WO 01/024793, WO 05/044299, WO02/081020, US2003/232884). In patent US2004/110833 ethyl pyruvate is used to influence cytokine mediated diseases. This is attributable to abolishing the effect of NF-kf3 (Han et al., 2005; Yang et al., 2004; Fink et al., 2004; Miyaji et al., 2003; Ulloa et al., 2002). However, opposite observations also exist in this respect (Mulier et al., 2005).

However, by no means these mechanisms indicate an inhibition of glyoxalases. Moreover, they can not be used to explain an inhibition of cell growth, because according to the findings of the present invention the growth of yeast cells is also inhibited by ethyl pyruvate, which cells neither have NF-kß nor cytokines nor other inflammatory mediators.

Additionally, it could be shown that protein adducts of methylglyoxal, the concentration of which is increased after inhibition of glyoxalases, even increase the release of TNF-a and the activation of NF-kß (Fan et al., 2003). In particular, this mechanism can not be used to explain the inhibitory effect of ethyl pyruvate on proliferation as the effect of ethyl pyruvate on cytokines is also detectable when cells are not proliferating.

The inhibitory effect of ethyl pyruvate on proliferation mediated via the inhibition of glyoxalases is the more surprising as ethyl pyruvate, due to its known effect as "scavenger" of reactive oxygen radicals should rather have a growth enhancing effect (Varma et al., 1998). As a matter of fact, this has been described for normal human T-lymphocytes (Dong et al., 2005). In this report it has furthermore been described that the formation of the cytokine interleukin-2 was enhanced in these cells.

### B. Pharmaceutical composition/manufacture of a medicament

The present invention relates to pharmaceutical compositions for topic administration comprising compounds according to formula (I) and glucose for the use in the treatment and/or prohylaxis of fungal infections.

In the following, particular embodiments will be described in the context of pharmaceutical compositions. However, it is to be understood that these embodiments also apply to the manufacture of a medicament. In other words, any disclosure of an embodiment in the context of a pharmaceutical composition is not to be understood as being limited thereto, but also relates to the manufacture of a medicament. Thus, the terms "pharmaceutical composition" and "use for the manufacture of a medicament" in the context of this application are interchangeable. This applies to the entirety of the present application.

In one embodiment the pharmaceutical composition can comprise a combination of one or more compounds of the general formula (I) wherein R3 and R4 together are =0, e.g. ethyl pyruvate, and one or more compounds wherein R3 or R4 is -OH like compounds of the general formula (II) and (III), e.g. ethyl lactate, (ethyl D- and/or L-lactate).

The pharmaceutical composition for use according to the invention can further comprise one or more additional pharmaceutically active ingredients. In the context of combinations with further active ingredients the low toxicity of the compounds of the present invention as well as their metabolites is of particular advantage.

As further pharmaceutical compounds, preferably chemotherapeutics, immunosuppressive agents, common agents against worms and fungi, antibiotics, substances favoring cell differentiation like transcription- and growth factors, inhibitors of glycolysis or substrates for glycolysis are used.

For example, a combination of a compound of the present invention, such as ethyl pyruvate with common antifungal agents, such as one or more selected from fluconazole, ketoconazole, clotrimazole, bifonazole, econazole, itraconazole, terbinafine, naftifine, amorolfine, amphotericin B, nystatin, and nikkomycin, miconazolemiconazole, oxiconazole, sulconazole, flucytosine , griseofulvin, and biocidal peptides including defensines is preferably used.

A preferred combination consists of compounds of the present invention and an inhibitor of glycolysis wherein the inhibitor of glycolysis interferes with glycolysis downstream of the triosephosphate isomerase reaction. The rationale of such a combination is to increase the concentration of triosephosphates from which methylglyoxal evolves parametabolically or paracatalytically, and thus, to improve the efficacy of therapy.

Particularly preferred is the combination of compounds of the present invention, in particular ethyl pyruvate or the corresponding thioester, and oxamate, an inhibitor of lactate dehydrogenase. Also particularly preferred is the combination of compounds of the present invention and an inhibitor of the glycerol aldehyde phosphate dehydrogenase, such as iodide acetate, and/or the lactate dehydrogenase inhibitor oxamate.

Furthermore, chemotherapeutics in the context of a standard chemotherapy which generally exist for example for carcinomas and sarcomas can be used. Some representative examples of standard chemotherapeutic agents are cyclophosphamide and doxorubicin for the treatment of breast cancer and leukemia, taxol for the treatment of ovary cancer, and 5-fluorouracil or cisplatin for sarcoma.

In addition to the compounds according to formula (I) glucose is applied which stimulates the metabolism of infectious organisms, such as bacteria, fungi or protozoa. In this manner advantageously the concentration of methylglyoxal is increased further and the efficacy of the compounds of formula (I) is increased further, resulting in an enhanced efficacy of the pharmaceutical composition.

A further aspect of the invention is the use of compounds of formula (I) and glucose in combination with known or novel genetic methods like siRNA and antisense nucleotides for the targeted inhibition of enzymes or proteins to increase the sensitivity of tumors (Nesterova and Cho-Chung, 2004).

The pharmaceutical composition or medicament can further comprise one or more auxiliary substances useful for the galenic formulations of drugs, including, but not limited to, fillers, flavouring agents, stabilizers and agents that prevent microbial growth in the pharmaceutical composition.

The pharmaceutical composition can be in any suitable galenic formulation, depending on the kind of disease to be treated and the chosen route of administration. The skilled person can readily select and prepare a suitable preparation on the basis of common general knowledge. Pharmaceutical compositions for use according to the invention can be prepared according to known methods e.g. by mixing one or more effective substances with one or more carriers, and forming of e.g. tablets, capsules, or solutions. Where appropriate, solutions can be e.g. encapsulated in liposomes, microcapsules or other forms of containment.

Examples of suitable formulations comprise aqueous solutions which can optionally be buffered, water in oil emulsions, oil in water emulsions, creams, ointments and formulations comprising any of the foregoing.

The invention encompasses a pharmaceutical composition prepared in the form of a sustained release or controlled release galenic formulation. Such formulations allow the targeted release in e.g. a certain location or a certain tissue or organ, and/or allow the sustained release over a defined period of time.

A pharmaceutical preparation can also be prepared by mixing the ester components of the compounds of formula (I) under conditions at which compounds of the general formula (I) are formed. The pharmaceutical preparation can also be prepared by assembling ester components of the compounds of formula (I) such that in the organism, for example in the acidic environment of the stomach, the compounds of the general formula (I), (II) or (III) are formed. Ester components are for example an alkanol like for example ethanol and an organic acid like for example lactic acid.

The pharmaceutical composition for use according to the invention comprises at least one compound of formula (I) in a therapeutically effective amount and glucose. The skilled person can readily determine the therapeutically effective amount in standard in vitro or in vivo experiments. For example, the effective amount can be estimated on the basis of an extrapolation from in vitro data, such as enzyme inhibition or cellular assays.

For example a dosage can be formulated in animal models which correspond to the IC50 in cell culture experiments. Hence, according to commonly known methods, the optimal dosage for the vertebrate to be treated, such as humans, can be deduced from animal experiments. The amount of the agent to be administered naturally depends on the person to be treated, his body weight, his genetic and physical constitution, the disease state, the route of administration, the galenic formulation and other parameters.

Furthermore, dosage and the interval of administration can be guided by the individual plasma concentrations of the agent that guarantee a therapeutic effect.

Useful effective concentrations, i.e. concentrations to be achieved at the level of cellular exposure, range from at least 0.05 mM, preferably from 0.05 mM to 50 mM, more preferably 1 mM to 40 mM, more preferably 1 mM to 20 mM, most preferably 1 mM, 2.5 mM, 5 mM, or 7,5 mM in systemic application. In topic applications higher concentrations may be useful. Preferred are 0.2 to 200 mM, more preferred are 0.2 to 50 mM and 50 to 200 mM.

In other words, the concentrations above refer to desired blood and/or tissue concentrations, or local concentrations. Thus, the invention relates to pharmaceutical preparations suitable to achieve such concentrations upon administration.

To achieve a therapeutic effect the pharmaceutical composition of the present invention is generally applied for several days or weeks as repeated bolus doses or continuous administration, or any time period required to achieve a therapeutic effect, at the respective therapeutically effective dosage.

The pharmaceutical composition of the present invention is administered topically. In topical administration a local administration to a selected site can be performed. Because of their nature, esters are of limited stability, necessitating the use of higher and/or repeated doses for systemic application. This can be circumvented by local topic application.

Pharmaceutical compositions for use according to the invention can be administered according to generally known methods - including but not limited to intraauricular, intranasal, percutaneous, or transdermal administration, or administration as an aerosol, via mini-pumps, as mouth lavage, gel, cream, oil, ointment, spray, plaster, via microbubbles and/or pulmonary application (e.g. by inhalation).

### C. Medical indications

The pharmaceutical composition or medicament of the present invention is for the following medical indications.

The invention encompasses the administration or use in invertebrates, non-mammalian vertebrates, mammals and humans in need thereof. In one embodiment the non-mammalian vertebrates are not fish. In the context of this application, the term "animal" is meant to encompass non-vertebrate animals, vertebrate animals, comprising non-mammalian vertebrates and mammals, which mammals comprise man. Thus, the term animal encompasses humans.

In particular, the pharmaceutical composition is for an animal, including man, suffering from a fungal infection, including the various specific examples of fungal infections discussed below.

According to the invention the cells proliferation of which is inhibited are fungi. Such fungi cause infectious diseases, such as various fungal diseases.

### a) Fungal infections

It was surprisingly found that the substances according to formula (I) can be used for the treatment and/or prophylaxis of fungal infections, and more specifically, inhibition and/or killing of fungi.

The term "treatment and/or prophylaxis of fungal infections" encompasses fungicidal and fungistatic effects.

The effects on glyoxalases, and the associated anti-fungal effects (i.e. fungistatic and/or fungicidal effects) of substances of the present invention have previously been unknown.

US 5,580,902 discloses certain substances used in the present invention as auxiliary agents in pharmaceutical compositions, in particular as enhancers for a multitude of active ingredients. This document does not disclose a fungicidal effect of substances used in the invention. WO 02/102366 describes certain pyruvate esters for the treatment of fish-parasites, such as plathelmintes.

US 2005/0020678 discloses substances, which are not encompassed by the substances of the present invention, as fungicides. It provides no teaching that could be generalized to other substances, however.

Other known medical applications of substances used in the invention equally provide no link to the treatment of fungal infections.

According to EP 0 717 984 and JP 8 208 422 proliferation of normal human cells, for example keratinocytes, is even stimulated by compounds of the present invention, which effect is used to improve the appearance of the skin. Similarly, in several patents (US 5,580,902; US 4,234,599; US 4,105,783) compounds used in the present invention have been described as agents to improve skin consistency and smoothen wrinkles. Effects on keratosis, and several diseases characterized by defective keratinisation (dandruff, acne, palmar and plantar hyperkeratosis, dry skin, Darier's disease, lichen simplex chronicus, psoriasis, eczema, pruritus, warts and herpes) are described in US 3,879,537, US 3,920,835, US 4,246,261 and US 7,33,815.

Moreover, ethyl lactate can be added to shampoos used in the treatment of canine superficial bacterial pyoderma (de Jaham, 2003). Ethyl pyruvate has been used to improve cataracts (Devamanoharan et al., 1999). In this connection a lowering of dulcitol and glycated proteins by ethyl pyruvate has been found, connected to the effect of pyruvate formed by hydrolysis of ethyl pyruvate. Moreover, methyl pyruvate has been suggested to treat fish parasites (WO 02/102366).

CN 1175632 describes ethyl lactate as an auxiliary substance in the manufacture of Spirulina wine, but does not disclose ethyl lactate as an active ingredient. WO 03/088955 and WO 02/074301 deal with reperfusion injury and inflammatory disorders. Marx et al (1988) suggests the inhibition of cancer cells by lactate. Stanko et al (1994), discusses a role of pyruvate in the treatment of cancer.

Though not intending to be bound by theory, it is suggested that the antifungal activity of the compounds used in the invention is due to their inhibition of glyoxalase I and/or II.

Living fungal cells generally require significant amounts of energy for cell division and general metabolism, provided in the form of ATP. Glycolysis allows anaerobic as well as, in combination with oxidative phosphorylation, aerobic energy generation.

Animals and humans regulate glucose concentration in their organs and body fluids in a narrow concentration range. The glucose concentration for example in human blood is constant at about 5 mM. Animals show a similar glucose homoeostasis.

Thus, most parasites, such as pathogenic fungi, are specialized in the utilization of glucose in blood, body cavities and on the skin, and show a high rate of glycolysis. Degradation of glucose by glycolysis and the formation of oxoaldehydes are ubiquitous metabolic pathways, which are phylogenetically highly conserved (Heymans and Singh, 2003; Clugston et al., 1997).

Thus, glycolysis per se has been discussed as a possible therapeutic target (Brady and Cameron, 2004; Kavanagh et al., 2004; Lakhdar-Ghazal et al., 2002, Iwami et al., 1995).

However targeting glycolysis for therapeutic purposes has so far remained elusive, as glycolysis is a central metabolic pathway of parasites as well their hosts.

US 2004/0167079 describes for example a method for treatment of cancer by use of 2-deoxyglucose (2-DG), an inhibitor of glycolysis.

US 2003/0181393 discloses the glycolysis inhibitors 2-deoxyglucose, oxamat and iodide-acetate. Iodide acetate inhibits glycerolaldehyde-3-phosphate dehydrogenase and oxamate inhibits lactate dehydrogenase.

The main shortcoming of inhibiting glycolysis is that glycolysis is used for energy generation in almost all cells, such that healthy cells are also affected by inhibition of glycolysis. In particular the influence on the brain is dramatic as the brain is an obligatory consumer of glucose and thus is highly dependent on glycolysis.

However, glycolysis is always accompanied by the formation of glyoxal compounds, in particular methylglyoxal. These compounds are highly toxic as they easily form adducts with cellular proteins and nucleic acids and lead to their inactivation.

Therefore, all glycolyzing cells use detoxification systems which are mostly consisting of the enzymes glyoxalase I and II.

An alternative approach to therapeutic intervention aiming at influencing modified and increased glycolysis is therefore no longer related to the inhibition of glycolysis, but to the inhibition of glyoxalases, which has extensively been discussed in the context of cancer (Thomalley et al., 1994; Vander Jagt et al., 1990; Pemberton and Barrett, 1989; Creighton et al., 2003; Hamilton and Batist, 2004 ).

Cells which cover their energy consumption exclusively by glycolysis in the presence of glucose are for example yeast cells. Similarly, pathogenic fungi, which can be treated according to the invention comprise, but are not limited to, Candida spp., Aspergillus spp., Cryptococcus spp., Pneumocystis spp., Zygomyces spp., Dermatophytes, Blastomyces spp., Histoplasma spp., Coccidoides spp., Sporothrix spp., Microsporidia spp., Malassezia spp. and Basidiomycetes, which also cover their energy consumption primarily via glycolysis when glucose is available in their human or animal hosts.

These organisms switch off other energy producing processes by glucose and use glycolysis (catabolite repression) or grow under hypoxic conditions.

As pathogenic fungi utilize glycolysis, as well as detoxifying glyoxalases, it is suggested by this invention that the inhibition of glycoxalases can serve as a "universal" therapy for a plurality of fungal infections.

The term "fungal infection" encompasses superficial colonisation by fungi, e.g. of the skin or mucosa as well as systemic infections. Also encompassed are infections of the gastrointestinal tract. The Pharmaceutical compositions are according to the invention for use in the treatment of mucosal (topic) diseases. The mucosal diseases can be caused by oral or vaginal infections. The oral or vaginal infections are for example the consequence of AIDS, chemotherapy or an immune suppressive therapy or immune suppressive conditions.

The term "treatment" encompasses subjects suffering from any of the various disease stages, such as acute or chronic infection, and encompasses after-treatment as well as prophylaxis. "After-treatment" means a treatment following conventional therapy. Treatment concomitantly with conventional therapy (e.g. known anti-fungal agents) is also part of the present invention.

The term "prophylaxis" or "chemo-preventivum" relates to administration of a pharmaceutical composition of the invention when a subject is at risk to develop a disease, or a disease is suspected or is present subclinically, but said disease has not fully evolved or has not been diagnosed.

The term "treating fungal infections" in the stricter sense relates to the treatment of clinically manifest disease. It is meant to encompass both cytotoxic and cytostatic effects. Thus, "inhibition of fungal cells" encompasses the inhibition of cell proliferation (fungistatic action) as well as the killing of the cells (fungicidal action). The killing of cells by necrosis or apoptosis is encompassed by the invention. The terms "proliferation" and "growth" are used interchangeably.

In one embodiment of the invention, the fungal infections are caused by a strain that is resistant to conventional antifungal agents, for example, fluconazole, ketoconazole, clotrimazole, bifonazole, econazole, itraconazole, terbinafine, naftifine, amorolfine, amphotericin B, nystatin, and nikkomycin, miconazolemiconazole, oxiconazole, sulconazole, flucytosine , griseofulvin, and biocidal peptides including defensins, in particular fluconazole. Thus, the invention in one embodiment relates to the treatment of fluconazole resistant Candida infections. Importantly, the compounds of the present invention also affect fungi that are resistant to conventional anti-fungal therapy, such as fluconazole resistant Candida, because they act via a different mechanism (Bennett et al, 2004).

### b) Use in the treatment of infections in immunosuppressed subjects

Fungal infections represent a significant problem in patients in an immunosuppressed state, and are amongst the leading cause of death, e.g. in transplant patients.

Thus, in one embodiment of the invention, the infection is in an immunosuppressed animal, wherein said immunosuppression is associated with hereditary or acquired immune-defects, comprising acquired immune defect associated with HIV, organ transplantation, chemotherapy or exposure to radiation.

Infections on an immunsuppressed background are oftentimes opportunistic infections by organisms that are non-pathogenic in the normal individual. Treatment of opportunistic infections is encompassed by the present invention.

### c) Use in the treatment of infections in cancer patients

Tumor patients are often in addition suffering from infectious diseases, due to a weakened immune defence, which results in a high sensitivity to infections.

Oftentimes cancer patients suffer from infectious disease caused by an ubiquitous, typically non-pathogenic organisms because of this weakened immune defence, also known as opportunistic infections.

It is therefore part of the invention to apply the pharmaceutical composition or medicament used in the invention for the treatment and/or prophylaxis against fungal infections, comprising opportunistic infections, in cancer patients.

As it is known that most tumors show a high rate of glycolysis (Gatenby RA and Gillies RJ, 2004), the growth of tumor cells and infectious agents, such as fungi can be simultaneously inhibited, which represents an increase in efficacy for the treatment of such patients. Thus, it is a particular advantage of the present invention that cancer cells, like fungal cells, exhibit an increased glycolysis, accompanied by high activity of glyoxalases. Hence, cancer cells can be targeted by the substances of formula (I), just like fungal cells. In other words, the substances used in the invention at the same time inhibit both kinds of cells.

Glyoxalase I is up-regulated in many tumors. Generally, it is presumed that increasing concentrations of glyoxalase I correlate with the malignant phenotype of tumors. The increased concentration of glyoxalase I in tumor tissue in comparison to normal tissue is said to increase the resistance of tumors to chemotherapeutics like mitomycin C and other anticancer agents (Ranganathan et al., 1995; Ayoub et al., 1993). Inhibition of the glyoxalase I reaction by compounds of the present invention, such as ethyl pyruvate, alone or in combination with conventional cancer therapy, such as radiation or chemotherapy is therefore advantageous for the treatment of cancer.

According to the invention, the animal can be concomitantly suffering from cancer, and can be going to receive, is currently receiving, or has received conventional cancer therapy, comprising one or more of chemotherapy, surgery, radiotherapy or brachytherapy. It is meant to encompass treatment following a completed conventional therapy (e.g. a full regimen of chemotherapy comprising several individual treatment periods, or following surgery), and treatment that is intermittent with the conventional therapy, e.g. taking place in the intervals between individual courses of chemotherapy. It is also meant to relate to a therapy that is started after the conventional therapy (e.g. after the first course of chemotherapy) and then continues concomitantly with the first therapy (e.g. throughout the further courses of chemotherapy).

Advantageously, the substances according formula (I) also are effective in cancer cells that are resistant against conventional therapy, such as chemotherapy and/or radiation therapy.

Moreover, the known effect of substances according formula (I), such as ethyl pyruvate as scavenger of reactive oxygen radicals represents a desired side effect for cells which do not have a high rate of glycolysis (non-cancer-cells) as such cells are additionally protected. In a combination therapy with a chemotherapeutic agent this is an additional advantage as also normal cells are stressed, which is reduced by compounds according formula (I).

In one embodiment the treatment of actinic keratoses with methyl- or ethylpyruvate is excluded.

The simultaneous inhibition of glyoxalases by compounds of the present invention such as ethyl or butyl pyruvate, ethyl or butyl lactate etc. in cancer cells as well as infectious organisms (in particular bacteria, fungi and protozoa) is particularly advantageous, as cancer cells and parasites are killed simultaneously.

### d) Use in the treatment of concomitant infectious disease

Fungal infections may be accompanied by other infections, such as bacterial infections or protozoal infections. Such multiple infections are of particular significance in immune-compromised individuals, and pose a significant clinical problem in transplant patients, cancer patients or HIV patients.

For example, the weakening of the patient by cancer per se, as well as by cancer therapy, such as chemotherapy, which weakens the immune system, favors fungal as well as bacterial infections. For such patients, even ubiquitous bacteria that are non-pathogenic for healthy human beings can be dangerous (opportunistic infections), the more so pathogenic bacteria.

Degradation of glucose by glycolysis and the formation of glyoxal compounds are ubiquitous metabolic pathways, which are phylogenetically highly conserved (Heymans and Singh, 2003; Clugston et al., 1997, Iwami et al, 1995). Cells which cover their energy consumption solely by glycolysis when glucose is available are for example yeast cells, but also cells of multicellular organisms like Helminthes (e.g. Schistosoma). They turn off other energy producing processes by glucose and use glycolysis (catabolite repression). It is therefore not surprising that many infectious organisms, such as fungi, bacteria and protozoa can also be inhibited in their growth by compounds according to formula (I).

Specific examples of infectious organisms which can be treated according to the invention comprise Trypanosoma, Leishmania, Plasmodium, Toxoplasma, helrnithes, Acrobacter, Actinobacillus, Actinomyces, Bacteroides, Brucella, Clamydia, Clostridium, Campylobacter, Escherichia, Enterobacter, Enterococcus, Eubacterium, Fusobacterium, Helicobacter, Hemophilus, Legionella, Listeria, Mycobacteria, Mycoplasma, Neissaria, Pasteurella, Peptostreptococcus, Pneumococcus, Pneumocystis, Porphyromonas, Prevotella, Pseudomonas, Salmonella, Shigella, Spirochetes, Staphylococcus, Streptococcus, Treponema, Vibrio, Yersinia, Escherichia coli or Pneumocystis carinii.

Importantly, the compounds according to formula (I) can also inhibit bacteria that are resistant against antibiotics, such as methicillin-resistant staphylococcus aureus (MRSA), which poses severe problems in the clinical setting (Cunha, 2005).

Prokaryotes including aerobic, anaerobic and facultative anaerobic bacteria, like fungi, perform glycolysis. Mechanisms exist in most of the bacteria even preferring the metabolism of the glucose (catabolite repression). Particularly in anaerobic organisms glycolysis is the most important way for generating energy.

Similarly, pathogenic protozoa, such as the bloodstream forms of trypanosoma, leishmania, plasmodium or toxoplasma, but also bilharzia, depend exclusively on glucose as energy source and undergo glycolysis.

In one embodiment of the invention, therefore, the animal, including man, is concomitantly suffering from a bacterial or protozoal infection as discussed above, including opportunistic infections and infections by organisms showing antibiotic resistance.

Therefore, it is a particular advantage of the present invention that fungal infections as well as bacteria and protozoa can be effectively treated with the compounds according to formula (I). Where in conventional therapies multiple active ingredients are required, the present invention provides substances that are effective against all these pathogens simultaneously. Consequently, the invention provides for a reduction in side effects, because only a single active ingredient is necessary, where the state of the art requires several different ingredients. In this context, the low toxicity of the compounds according to formula (I) is a further particular advantage.

### e. "Postprandial state"

In one embodiment the pharmaceutical composition or medicament is for use in a vertebrate having a reduced blood glucose level. In postprandial states the utilization of glucose is reduced in normal cells. These states can be reached for example by long-term fasting and can be accelerated by administration of hormones or can be forced by administration of hormones. Characteristic for such states is a low blood level of glucose and a high blood level of ketone bodies. Ketone bodies can be used by the brain to generate energy such that metabolic states of the patient can be generated under control of a medical practitioner prior to therapy wherein infectious organisms and/or tumors represent the primary consumers of glucose, under conditions of reduced blood glucose levels (Sugden and Holness, 2002). Thus, in a postprandial state the selectivity of the therapy is enhanced, because of the reduced glucose metabolism in normal cells.

### D) Anti-fungal applications

### a) Biofilms

The present invention comprises pharmaceutical compostions for use in the treatment and/or prophylaxis of a fungal infection in an animal, including humans by prevention of the formation of biofilms, as well as combating biofilms.

Fungi, like bacteria can produce biofilms after attachment to a surface, which is a mixture of cells that coexist as an organized community. Biofilms represent a protective environment and thus biofilm formation carries important consequences, both in industrial and clinical settings.

Sessile fungal cells within the biofilm are resistant to a range of antifungal agents currently in clinical use, such as triazoles and polyenes. Biofilms can stick to plastic, are able to coat medical implants causing serious complications in patients with hip and valve replacements, shunt tubing weavers and contact lens weavers, vascular bypass crafts and urinary catheter (Reynolds TB, and Fink GR. Science 2001).

Microbes, such as bacteria, fungi, protozoa, or nematodes are found in dental unit waterlines. The presence of various microorganisms is a potential source of microbial contamination of dental aerosols, and thus a potential threat to the health of patients and dental staff. In particular under conditions of decreased immunity of an organism, opportunistic infections constitute a health risk (Szymanska J. Ann Agric Environ Med. 2005)

The presence of microbes in drinking water and within biofilms of water distribution systems are associated with taste and odor problems, contamination in food and beverage preparation, and a variety of health-related effects (Doggett MS. Appl Environ Microbiol. 2000).

Biofilms containing microbes can be dangerous in space stations. In long-term missions fungi formation in closed systems can affect the astronauts' health and water-thin biofilms can attack inaccessible cable harnesses causing electric breakdown.

Microorganisms have been implicated in the attack of both natural limestone materials and concrete. For example, the fungus Fusarium plays an important role in concrete deterioration. Treatment of microbial growth as constituents of biofilms is therefore promising for the protection of historical buildings and gravestones. (Gu, et al, International Biodeterioration & Biodegradation. 1998).

Microbial spoilage is a constant concern in the food processing industry. Even breweries have a risk of contamination of their products, even though there is a predisposition of beer per se to be contaminated with bacteria due to low pH-value (3.8-4.7). Most important beer-spoiling organisms belong to the genera Lactobacillus, Pediococus, Pectinatus, and to yeast representing Saccharomyces or Dekkera. Beer-spoiling bacteria are facultative or obligate anaerobes and are acidophilic or at least acidotolerent.

Biofilms have been found in brewery pasteurizers and conveyor systems. Bacteria associated with biofilms with conveyor tracks and bottles and can warmers belong to Pseudomonas, Enterobacter, Bacillus, and to yeast representing Saccharomyces, Candida, Rhodotorula and others (Storgards E, et al, J. Am. Soc. Brew. Chem. 2006).

In the context of the present invention the term "biofilm" relates to microorganisms including fungi attached to a substrate by means of an extracellular matrix produced by said bacteria, and comprising said bacteria. Biofilms can anchor microorganisms to substrates formed by medical material and living substrates such as teeth. Thus, the invention relates to the prevention and or combating biofilms on such kinds of substrates.

In one embodiment, the substrates are teeth, and the biofilm may optionally be associated with the formation of dental plaque.

It is of particular advantage in this connection that substances of the invention not only affect fungi, but also other organisms, such as e.g. bacteria and protozoa. Biofilms are typically colonized by other organisms in addition to fungi. Thus, the substances of the present invention can influence (i.e. inhibit their growth, and/or exert cytostatic and/or cytotoxic effects) many organisms typically found in biofilms (Armitage,2004; Leclerc, 2005; Coetser and Cloete, 2005; Chandra et al., 2005).

Moreover, the present invention relates to the treatment of infections associated with biofilms, such as infections caused by the inhalation of fragments of biofilms (e.g. inhaled biofilm fragments derived from contaminated inhalation devices, such as in dental unit waterlines or others).

It is a further particular advantage of the substances according formula (I) that they can attack biofilms, which are difficult to combat with conventional antibiotics.

On the basis of the following figures and examples the present invention is illustrated further.

### BRIEF DESCRIPTION OF THE FIGURES

Fig. 1: Inhibition of the enzymatic activity of glyoxalase I of yeast by ethyl pyruvate (EP).
Fig. 2: Inhibition of the enzymatic activity of yeast glyoxalase I by 2-Oxopropanethioic acid S-ethyl ester (SE).
Fig. 3: Influence of ethyl pyruvate (EP), butyl pyruvate (BP) and butyl L-lactate (BL) on the enzymatic activity of yeast glyoxalase I.
Fig. 4: Influence of ethyl pyruvate (EP) on the enzymatic activity of yeast glyoxalase II.
Fig. 5: Inhibition of the enzymatic activity of glyoxalase I of human erythrocytes by ethyl pyruvate (EP).
Fig. 6: Inhibition of the enzymatic activity of glyoxalase II of human erythrocytes by ethyl pyruvate (EP).
Fig. 7: Effect of ethyl D-lactate (DEL), ethyl L-lactate (LEL) and ethyl pyruvate (EP) on the vitality of primary human fibroblasts.
Fig. 8: Inhibition of growth of yeast cells by ethyl pyruvate (EP).
Fig. 9: Effect of ethyl pyruvate (EP) on growth of different microbial species.

### EXPERIMENTS

### A. General experimental procedures

### a) Determining enzymatic activity of yeast glyoxalase I

Measuring glyoxalase I (E.C.4.4.1.5, lactoyl-glutathione lyase Sigma, G-4252) was performed according to the instructions of McLellan and Thornalley (1989). The principle is based on measuring the initial rate of formation of S-D-lactoyl-glutathione from methylglyoxal (Sigma, M0252, 40%) and reduced L-glutathione (Aldrich, G-4251, >99%). The formation of the product is observed using the extinction coefficient e = 2,86 mM -1 cm -1 at 240 nm. The measurement was performed in 50 mM sodium phosphate buffer, pH 7,0. For that purpose 2 mM methylglyoxal and 2 mM reduced glutathione were incubated for 2 minutes at 30°C for the formation of the hemithioacetal. Thereafter 20 µl of a 1 to 1000 dilution of the glyoxalase I (Sigma, G-4252) was added to 1 ml of the measuring reagent to start the reaction.

Glyoxalase activity (IU) corresponds to the amount of enzyme forming 1 µmol of S-D-lactoyl-glutathione/min.

### b) Determining enzymatic activity of human erythrocytes glyoxalase I

The determination of glyoxalase I was performed as described above for the yeast enzyme. After the formation of the hemithioacetal, suitable amounts (5 to 100 µl) of an erythrocyte lysate were added to 1 ml of the measuring reagent to start the reaction. The erythrocyte lysate was prepared according to the instructions of Mannervik et al. (1982).

Glyoxalase activity (IU) corresponds to the amount of enzyme forming 1 µmol of S-D-lactoyl-glutathione/min.

### c) Determining enzymatic activity of human erythrocyte glyoxalase II

The determination of erythrocyte glyoxalase II (E.C.3.1.2.6., hydroxyacyl glutathione hydrolase) was performed according to the instructions of McLellan and Thomalley (1989). The principle is based on determining the initial hydrolysis of S-D-lactoyl-glutathione (L 7140, Sigma - Aldrich Chemie GmbH) in the presence of low amounts of a cell extract. Hydrolysis is observed on the basis of the reduction of the extinction at 240 nm (e = -3,1 mM -1 cm -1). Glyoxalase II activity (IU) correlates to the amount of enzyme hydrolysing 1 µmol of S-D-lactoyl-glutathione/min. The measurement was performed in 100 mM Tris-HCl buffer, pH 7,4. For this purpose 0.4 mM S-D-lactoyl-glutathione was added to 1 ml of the measuring reagent and the reaction was started by addition of suitable amounts (5-100 µl) of an erythrocyte hemolysate. The erythrocyte lysate was prepared according to the instructions of Mannervik et al. (1982).

### d) Protocol for experiments using Saccharomyces cerevisiae HD 65-5a

A colony of strain HD 65-5a (Saccharomyces cerevisiae) was inoculated in 5 ml YPD-medium [(2% glucose (Fluka), 1 % yeast extract (BD, Sparks), 2% peptone (BD, Sparks)] in a rotating 20 ml glass tube and was incubated overnight at 30°C. At an O.D. of 12,9 (580 nm) a starting density of O.D. 0,19 was adjusted by dilution with a culture medium.

The culture was aliquoted to 10 ml (in 20 ml glass tubes), and the respective test substances were added. The aliquots were incubated at 30°C under continuous rotation of the tubes (200 U/min). At the indicated times, samples were removed and optical density was determined at a wavelength of 600 nm.

### e) Protocol for experiments using LNCaP cells (androgen dependent prostate carcinoma cells; DSMZ No ACC 256)

An identical number of LNCaP cells (androgen dependent prostate carcinoma cells; DSMZ No ACC 256) were routinely cultured in 75 cm² culture flasks in RPMI-1640 medium (Gibco; Nr. 21875-034), penicillin/streptomycin (100 units penicillin/ml; 100 µg streptomycin/ml; Gibco; Nr. 15140/122) in the presence of 10% fetal calf serum (Biochrom; Nr. S0113/5; RPMI-FKS). The flasks were incubated at 37°C in a humidified atmosphere (relative humidity >95%) of 5% CO₂ in air. After reaching 50% confluency the medium was removed and the adherent cells were washed twice with PBS (phosphate buffered sodium chloride; 50 mM sodium phosphate, 150 mM NaCl, pH 7,4). Thereafter, the cells were incubated with serum free RPMI-medium (RPMI-SF) comprising the experimental supplements in five flasks each (i.e. five replicates each). The culture was continued at 37°C, 5% CO2 and 95% humidity for 24 hours. Thereafter the respective supernatants were removed and the adherent cells were detached from the bottom of the plate by trypsin/EDTA (Gibco; No. 25300-054) and were pelleted. After resuspension and homogenization the cells were counted using a hemocytometer.

### B. Data

### Example 1

### Inhibition of the enzymatic activity of glyoxalase I of yeast by ethyl pyruvate (EP).

Determination of glyoxalase I activity was performed according to the general protocol as described above. The influence of ethyl pyruvate on enzyme activity was investigated by addition of increasing concentrations of EP (Sigma; no. E4,780-8; lot. S18972-513) to the measuring reagent.

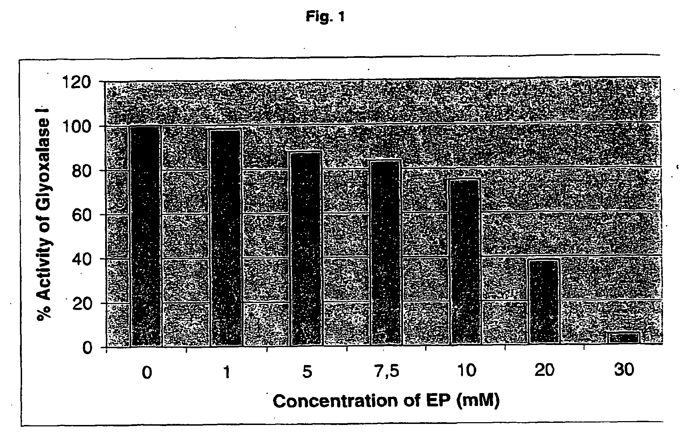

The experiments (Fig. 1) show that EP inhibits the reaction of yeast glyoxalase I in a concentration dependent manner.

### Example 2

### Inhibition of yeast glyoxalase I by compounds of the general formula (I), (II), and (III), respectively

Determination of glyoxalase I activity was performed according to the general protocol as described above. The influence of compounds of the general formula (I), (II), and (III) on the activity of the enzyme was investigated by addition of increasing concentrations of these compounds to the preparation. The IC50-values were calculated from inhibition curves of each compound.

The data (Table 3) show that alkyl 2-oxo-derivatives inhibit the enzymatic activity of yeast glyoxalase I with different IC₅₀s, while the alkyl 2-hydroxy-derivatives revealed no inhibitory effect at all. Thus, the experiments demonstrate that alkyl 2-hydroxy-derivatives are prodrugs which must be activated to the respective 2-oxo-derivatives in living cells or organisms.

**Table 3**

| Alkyl 2-Oxo-Derivatives | | IC₅₀ GLOI* |
|---|---|---|
| Ethyl 2-oxobutyrate | CH3-CH2-O-CO-CO-CH2-CH3 | 1,8 mM |
| Butyl pyruvate | CH3-CH2-CH2-CH2-O-CO-CO-CH3 | 7,5 mM |
| Ethyl pyruvate | CH3-CH2-O-CO-CO-CH3 | 10 mM |
| Methyl pyruvate | CH3-O-CO-CO-CH3 | 15 mM |
| Diethyl oxalate | CH3-CH2-O-CO-CO-OCH2-CH3 | >50mM |
| | | |

| Alkyl-2-Hydroxy Derivatives | | |
|---|---|---|
| (-)-Butyl L-Lactate | CH3-CH2-CH2-CH2-O-CO-CHOH-CH3 | no inhibition |
| (-)-Ethyl L-Lactate | CH3-CH2-O-CO-CHOH-CH3 | no inhibition |
| (+)-Ethyl D-Lactate | CH3-CH2-O-CO-CHOH-CH3 | no inhibition |
| Methyl L-Lactate | CH3-O-CO-CHOH-CH3 | no inhibition |
| (-)-Isopropyl L-Lactate | CH3-CH(CH3)-O-CO-CHOH-CH3 | no inhibition |
| (+)-isobutyl L-Lactate | CH3-CH(CH3)-CH2-O-CO-CHOH-CH3 | no inhibition |

| | | |
|---|---|---|
| *GLO I = glyoxalase I | | |

### Example 3

### Effect of prodrugs

The compounds of the general formula (II) and/or (III) can act as prodrug in the sense that the compounds are activated by enzymes within cells or in the organism, or are oxidized in vitro by addition of a suitable oxidant.

In cells or organisms lacking such activation systems the compounds of the general formula (II) and/or (III) remain inefficient. This is demonstrated by the effect of EP and LEL on the activity of glyoxalase I and the proliferation of tumor cells as well as yeast cells.

**Table 4**

| | Inhibition of enzyme | Inhibition of cell proliferation | |
|---|---|---|---|
| | yeast GLO I | LNCaP | Yeast cells |
| Ethyl pyruvate | + | + | + |
| Ethyl L-lactate | - | + | - |

The experiment (Table 4) shows that alkyl 2-hydroxy derivatives have to be activated into alkyl 2-oxo-derivatives by endogenous activation systems to inhibit cell proliferation via inhibition of GLO1. In contrast to human tumor cells (LNCaP), yeast cells lack enzyme systems for transformation of alkyl 2-hydroxy derivatives into alkyl 2-oxo-derivatives and therefore proliferation can not be inhibited by alkyl 2-hydroxy derivatives directly.

### Example 4

### Inhibition of the enzymatic activity of yeast glyoxalase I by 2-Oxopropanethioic acid S-ethyl ester (SE).

### Synthesis of 2-Oxopronanethioic acid S-ethyl ester

In a 500 ml three necked flask equipped with a reflux condenser and dropping funnel N, N'-dicyclohexylcarbodiimide (20.6 g, 0.1 mol; Cat.No. 36650, Lot. RA 13160, Fluka, Germany) was dissolved in dry tetrahydrofuran (200 mL; Cat.No. AE 07.1, Lot. 2121/5CR, Roth, Germany). Then, a solution of ethanethiol (6.2 g, 0.1 mol; Cat.No. EC 200-837-3, Lot. A0200018001, Acros Organics) in tetrahydrofuran (25 mL) was added. Under stirring a solution of 2-oxopropanoic acid (8.9 g, 0.1 mol) in tetrahydrofuran (25 mL) was added dropwise within 10 min without external heating. The solution warmed up to 45°C, turned yellow and a colourless precipitate of N,N'-dicyclohexylurea appeared. After complete addition the suspension was heated to reflux for 3 min and then cooled to 0° C. The urea was filtered off and the solvent removed from the filtrate by distillation. Without other manipulations the remaining orange oily residue was rapidly distilled in the air stream of a heat gun at normal pressure yielding a yellow fraction between boiling point (bp) 145 and 165 °C at normal pressure (amount of crude product 4.0 g, n_{D} 1.4802 (21 °C)) together with a considerable amount of brown resin in the distillation flask. The yellow crude product was redistilled in the same manner to yield 2.3 g of 2-oxopropanethioic acid S-ethyl ester as yellow oil, bp 153-157 °C, nD 1.4750 (21 °C), purity > 95 % (NMR).
1H-NMR (300.06 MHz, CDCl3): □ 1.29 (-CH2-CH3), 2.41 (H3C-CO-), 2.92 (-CH2-CH3); 13C-NMR (75.45 MHz, CDCl3): □ 14.2 (-CH2-CH3), 23.2 (-CH2-CH3), 23.9 (H3C-CO-), 191.4 (-CO-), 193.4 (-CO-). NMR spectra were measured with a Varian Mercury-300BB spectrometer. Chemical shifts are reported at the □ scale in ppm.

### Determination of glyoxalase I activity

Determination of glyoxalase I activity was performed according to the general protocol as described above. The influence of 2-oxopropanethioic acid S-ethyl ester on the activity of the enzyme was investigated by addition of increasing concentrations of SE to the preparation for the measurement.

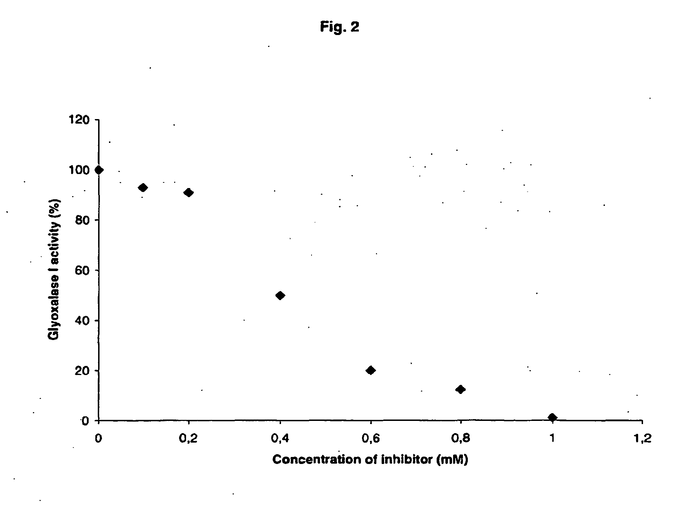

The experiment (Fig. 2) shows that SE inhibits the reaction of the glyoxalase I of yeast in a concentration dependent manner.

### Example 5

### Influence of ethyl pyruvate (EP), butyl pyruvate (BP) and butyl L-lactate (BL) on the enzymatic activity of yeast glyoxalase I.

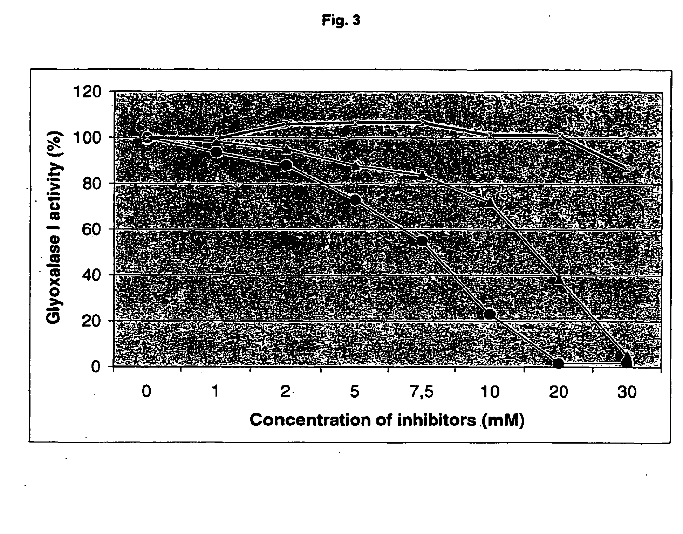

Determination of glyoxalase I activity was performed according to the general protocol as described above. The influence of effectors on the enzymatic activity was investigated by addition of increasing concentrations (0 - 30 mM) of EP (Sigma; no. E4,780-8; lot. S18972-513) (triangle), BP (prepared according to the instructions of patent JP 11080089; 98 %) (circle) and BL (Fluka, 69819; lot. 443090/1 21503090) (squares) to the measuring reagent (Fig. 3).

The experiments show that EP (triangles) as well as BP (circles) inhibit the activity of glyoxalase I in a concentration dependent manner wherein the effect of BP is stronger than the effect of EP. BL (squares) does not influence the reaction of glyoxalase when it is not transformed into BP.

### Example 6

### Influence of ethyl pyruvate (EP) on the enzymatic activity of yeast glyoxalase II.

A colony of strain HD65-5a (Saccharomyces cerevisiae) was incubated in 5 ml YPD-medium [(2% glucose (Fluka), 1% yeast extract (BD, Sparks), 2% peptone (BD, Sparks)] over night at 30°C under rotation. A 10 ml aliquot was added to 200 ml culture medium in a 500 ml glass flask and was incubated at 30°C on a shaker (250 U/min). The yeast cells were harvested in the stationary growth face (O.D. 1cm/600nm = 2-4) by centrifugation (15 min, 3000 x g). The cells were diluted with 0.1 M MES buffer, pH 6,5 to an O.D. of 4 and were then disrupted in a glass mill (Schwock et al., 2004). Subsequently the disrupted cells were centrifuged at 23000 x g, 4°, 30 min. Protein concentration of the cell free extract was determined according to the Bradford method (Bradford, 1976).

The activity of the glyoxalase II (Hydroxyacyl glutathione hydrolase, E.C. 3.1.2.6.) was determined according to the instructions of Martins et al. (1999) in 0.1 M MES buffer, pH 6,5, 1.5 mM S-D-lactoyl-glutathione (L7140, Sigma-Aldrich Chemistry GmbH) and 0.75 mM DTNB (Sigma, D8130). After addition of suitable amounts of cell extract and an incubation period of 15 min at 25°C the formation of glutathione was measured at 412 nm (□ =13.6 mM⁻¹ cm ⁻¹). The activity of the glyoxalase II (IU) correlates with the amount of enzyme hydrolysing 1µmol of S-D-lactoyl-glutathion/min.

The influence of ethyl pyruvate on the activity of the enzyme was investigated by adding increasing concentrations of EP (Sigma, No. E4,780-8; Lot. S18972-513) (0-20mM) to the measurement reagent.

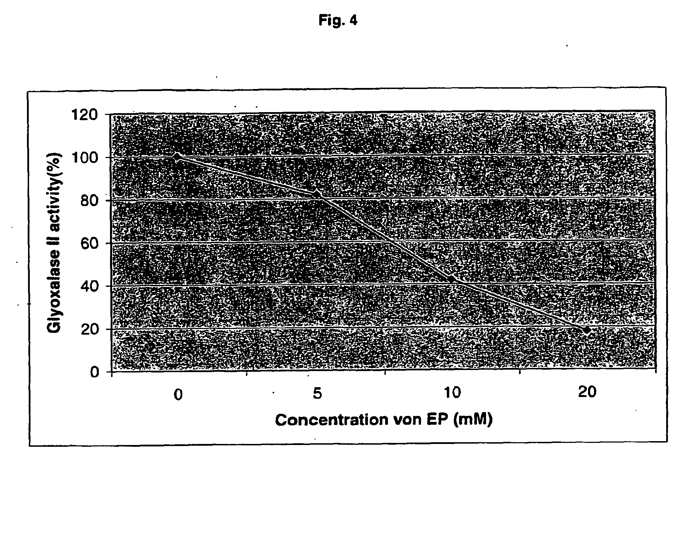

The relative activities of glyoxylase II in presence or absence of EP are illustrated in Fig. 4. The experiment shows that EP inhibits the reaction of yeast glyoxalase II in a concentration dependent manner.

### Example 7

### Inhibition of the enzymatic activity of glyoxalase I of human erythrocytes by ethyl pyruvate (EP).

Determination of glyoxalase I activity was performed according to the general protocol as described above. The influence of ethyl pyruvate on enzyme activity was investigated by addition of increasing concentrations of EP (Sigma; no. E4,780-8; lot. S18972-513) (0-50mM) to the measuring reagent.

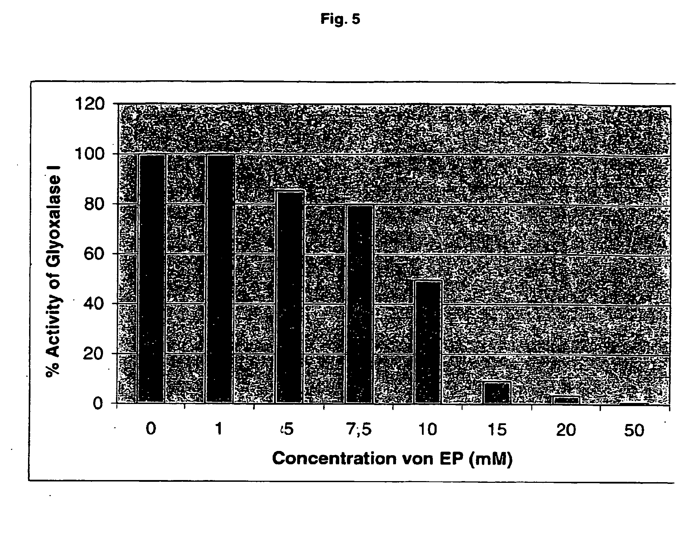

The experiment (Fig. 5) shows that EP inhibits the reaction of glyoxalase I of human erythrocytes in a concentration dependent manner.

### Example 8

### Inhibition of the enzymatic activity of glyoxalase II of human erythrocytes by ethyl pyruvate (EP).

Determination of glyoxalase II activity was performed according to the general protocol as described above. The influence of ethyl pyruvate on the activity of the enzyme was investigated by addition of increasing concentrations of EP (Sigma, no. E 4,780-8; lot. S18972-513) (0-20 mM) to the measuring reagent.

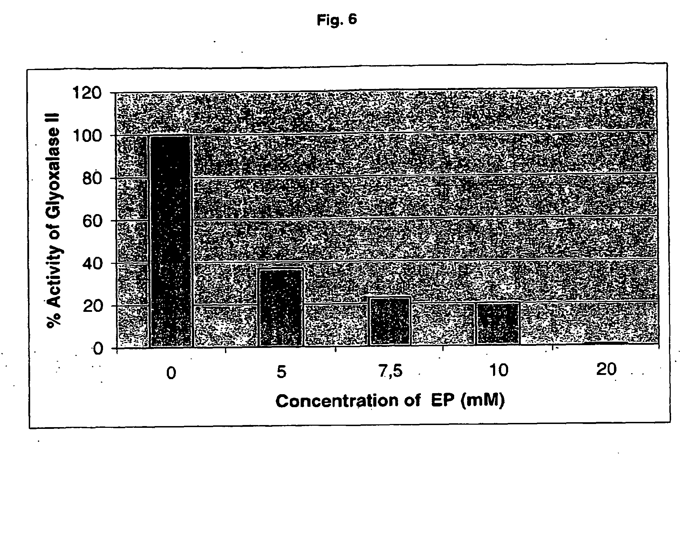

The experiment (Fig. 6) shows that EP inhibits the reaction of glyoxalase II in a concentration dependent manner.

### Examples 9

### Effect of ethyl D-lactate (DEL), ethyl L-lactate (LEL) and ethyl pyruvate (EP) on the vitality of primary human fibroblasts.

An identical number (104 cells per well) of primary human skin fibroblasts were inoculated in 24-well plates (Greiner, no. 662160) and were cultured in DMEM (Gibco; no. 41966-092) in the presence of 10 % calf serum (Biochrom, S0113/5), 2 mM L-glutamine (Gibco; no. 25030-024), penicillin/streptomycin (100 units penicillin/ml; 100 µg streptomycin/ml, Gibco; no. 15140/122), 5 mg% ascorbic acid (Serva, no. 14030.02) at 37°C, 5%CO2 and 95% humidity. The primary human fibroblasts were prepared according to the instructions of Birkenmeier et al. (1998). After reaching 50 % confluency the medium was by fresh serum free medium. Thereafter the following supplements were added to the cells: preparation 1 (equivalent volume of serum free (SF) medium, blank); preparation 2 (1 mM DEL or 1 mM LEL or 1 mM EP); preparation 3 (5 mM DEL or 5 mM LEL or 5 mM EP), preparation 4 (10 mM DEL or 10 mM LEL or 10 mM EP), preparation 5 (20 mM DEL or 20 mM LEL or 20 mM EP), preparation 6 (50 mM DEL or 50 mM LEL or 50 mM EP). The culture was continued at 37°C, 5% CO2 and 95% humidity for 24 hours. Thereafter the supernatants were removed and 100 µl of a 50 % thymol blue solution was added to the wells. After washing the cells with medium the unstained and stained cells were counted under the light optical microscope comprising a coordinate plane. Cells stained blue were assessed as avital, unstained cells as vital. The percentage of unstained cells of the total number of cells corresponds to the vitality of the cells.

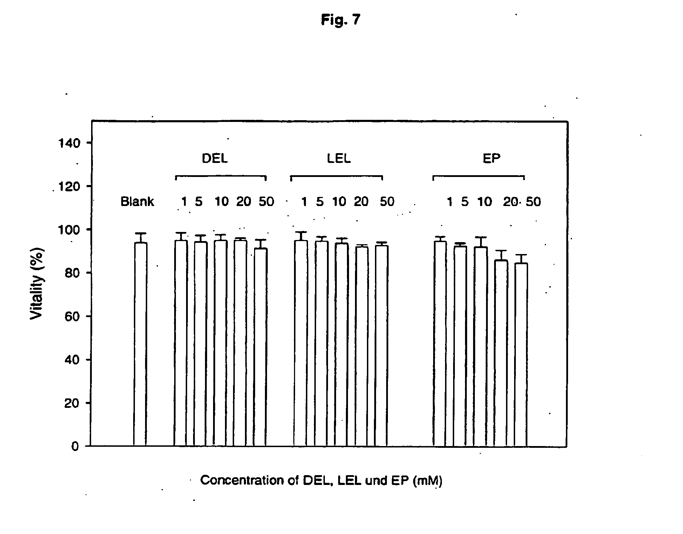

The experiment (Fig. 7) shows that DEL, LEL and EP are not toxic over the concentration range investigated and that they do not significantly influence vitality of primary human fibroblasts.

### Example 10

### Inhibition of growth of yeast cells by ethyl pyruvate (EP).

A colony of Saccharomyces strain HD65-5a was cultured as describe above.

Increasing concentrations of ethyl pyruvate were added and the culture continued as described above. After defined intervals 1 ml samples were removed and the O.D. was measured at 600 nm.

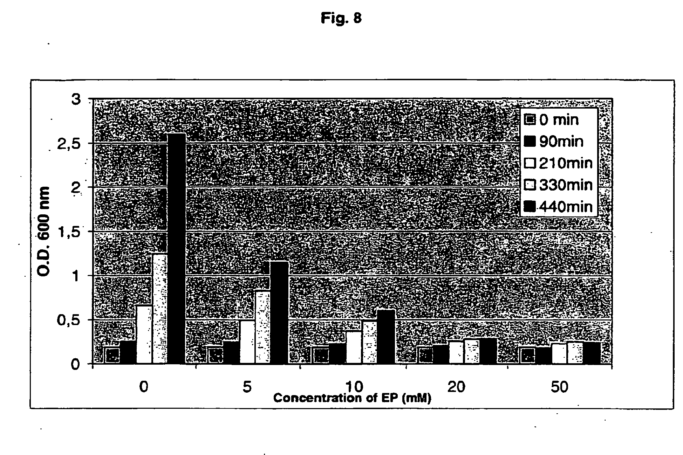

The experiment (Fig. 8) shows that EP inhibits the growth of yeast cells in a concentration dependent manner.

### Example 11

### Inhibition of the growth of yeast cells (Saccharomyces cerevisiae) by ethyl pyruvate (EP), butyl pyruvate (BP), ethyl L-lactate (LEL) and oxidized ethyl L-lactate (LEL).

A colony of Saccharomyces strain HD65-5a was cultured as describe above. Increasing concentrations of EP, BP and LEL (without pre-treatment) were added. LEL, which had been oxidized with potassium permanganate (KMn04), was added to another aliquot. Samples of 1 ml were taken after 8 hours and were measured at 600 nm.

The experiment (Table 5) shows that EP and BP inhibit the growth of yeast cells in a concentration dependent manner. LEL did not influence growth, whereas in contrast, oxidized LEL affects proliferation.

**Table 5:**

| | Concentration of the inhibitor | | | | |
|---|---|---|---|---|---|
| Inhibitor | without | 1 mM | 10 mM | 20 mM | 50 mM |
| | Number of cells in [%] of the total cell number in the sample without inhibitor | | | | |
| Ethyl pyruvate (EP) | 100 | 94 | 23 | 11 | 9.4 |
| Butyl pyruvate (BP) | 100 | 96 | 88 | 73 | 14 |
| Ethyl L-lactate (LEL) + KMnO₄ | 100 | 101 | 94 | 75 | 62 |
| Ethyl L-lactate (LEL) | 100 | 102 | 98 | 91 | 95 |

### Example 12

### Inhibition of a fluconazole resistant strain of Candida albicans by ethyl pyruvate (EP), butyl pyruvate (BP), and ethyl 2-oxobutyrate (EOB).

A colony of the fluconazole resistant Candida albicans strain A435 5 was cultured in the same medium and under the same conditions as used for Saccharomyces HD65-5a.

At an O.D. of 14.4 (600 nm) a starting density of O.D. 0.20 was set by dilution with culture medium. The preparation was aliquoted to 10 ml (in 20 ml glass tubes), increasing concentrations of EP, BP and EOB were added and were incubated at 30°C with continuous rotation of the tubes (200 U/min).

Samples of 1 ml were taken after 5 hours and the O.D. at 600 nm was determined as a standard for the cell number. The resistance against fluconazole was determined according to the guidelines of NCCLS (1997). Resistance is defined as growth at a dosage of ≥ 64 µg/ml of fluconazole.

The experiment (Table 6) shows that EP, BP and EOB inhibit the growth of fluconazole resistant Candida albicans cells.

**Table 6**

| | Concentration of the inhibitor | | | | |
|---|---|---|---|---|---|
| Inhibitor | without | 1 mM | 10 mM | 25 mM | 50 mM |
| | Number of cells in [%] of the total cell number in the sample without inhibitor | | | | |
| Ethyl 2-oxobutyrate (EOB) | 100 | 81 | 5,7 | 1,4 | 1,7 |
| Ethyl pyruvate (EP) | 100 | 94 | 1.8 | 0.9 | 0.9 |
| Butyl pyruvate (BP) | 100 | 100 | 42 | 15 | 7 |

### Example 13

### Inhibition of the growth of a fluconazole sensitive strain of Candida albicans by ethyl pyruvate (EP) and butyl pyruvate (BP).

A colony of the fluconazole sensitive Candida albicans strain R/H013 was cultured as described above.

After aliquoting, increasing concentrations of EP and BP were added and were incubated at 30°C under continuous rotation of the tubes (200 U/min). Samples of 1 ml were taken after 9 hours and the O.D. was measured at 600 nm. The strain was sensitive against fluconazole at a dosage of ≤ 0.13 µg/ml.

The experiment (Table 7) shows that EP and BP inhibit the growth of fluconazole sensitive Candida albicans cells.

**Table 7**

| | Concentration of the inhibitor | | | | |
|---|---|---|---|---|---|
| Inhibitor | without | 1 mM | 10 mM | 25 mM | 50 mM |
| | Number of cells in [%] of the total cell number in the sample without inhibitor | | | | |
| Ethyl pyruvate (EP) | 100 | 89 | 3 | 1 | 1 |
| Butyl pyruvate (BP) | 100 | 100 | 55 | 15 | 7 |

### Example 14

### Effect of ethyl pyruvate (EP) on growth of different microbial species

The agar dilution method was used for antimicrobial susceptibility test. A series of plates (petri dishes; Greiner, CatNo. 933161) were prepared with 20 ml agar medium (Iso-Sensitest-Agar Ca.No. CM474, OXOID, Germany) to which various concentrations of EP (12.5mM to 40 mM) were added. Control agar plates were prepared without EP. The plates are then inoculated with a suitable standardized suspension of the test microbial species (10⁴ cfu) using an automated spotter (spots 1-21). The agar plates were incubated under aerobic conditions at 37°C for 20 hours. At the respective positions of the spots growth (if permissive under the selected conditions) will result in the formation of cell discs. The evaluation of the test results is performed by inspecting the sizes of these discs.

The following microbials were spotted to the agar dilution: (1) Candida albicans ATCC 90028 (2) Candida albicans (Patient isolate), (3) Candida krusei, ATCC 6258, (4) Candida krusei (Patient isolate), (5) Candida tropicalis (Patient isolate), (6) Candida glabrata (patient isolate), (7) Candida parapsilosis, ATCC 22019, (8-21) prokaryotic controls.

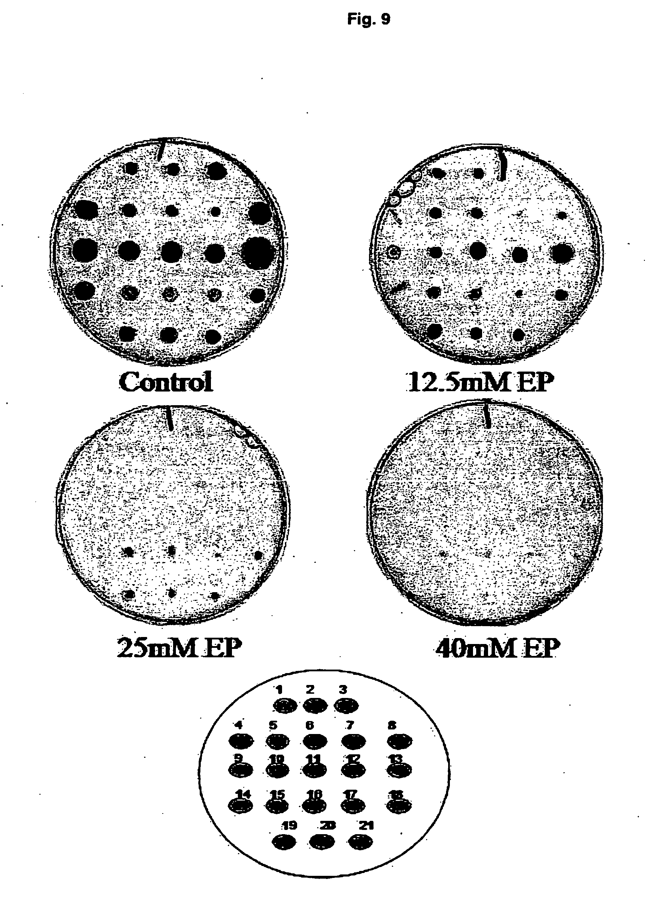

The experiment (Fig. 9) shows that EP effectively inhibits the growth and proliferation of different fungi (1-7).

### Example 15

### Mouth lavage in a patient suffering from candiasis

A male patient at the age of 54 with a diagnosis of diabetes mellitus (presenting with a fasting blood sugar level of 6.88 mM) complained about dryness in the mouth, chapped lips and white blotchy alterations of the oral mucosa. An oral smear test showed an infection with Candida albicans. Determination of the germ number in the lavage of the oral cavity (10 ml physiological sodium chloride solution, 30 seconds) showed a value of > 100 CFU/ml of lavage.

The patient treated his mouth 3 times daily with a lavage of 10 ml ethyl pyruvate solution (75 mM ethyl pyruvate, 1 % glucose in physiological sodium chloride solution) over 4 days. A further determination of the number of germs after a lavage with 10 ml sodium chloride solution showed a reduction of germ number to < 5%.

The results show that the application of ethyl pyruvate is helpful for oral candidiasis.

### containers and stored at

### Reference example 16:

### Cream

A cream comprising a substance according to formula (I) is prepared from the following ingredients:

| | | |
|---|---|---|
| aqueous phase: | butyleneglycol | 4% |
| | substance of formula (I) | 25% |
| | water | to 100% |
| lipid phase: | steareth-2 | 3% |
| | steareth-21 | 2% |
| | glycol-15-stearyl ether | 9% |
| | cetearylalcohol | 2,5% |
| therafter addition of: | | |
| | phenoxyethanol, methylparaben, | |
| | ethylparaben, propylparaben, | |
| | butylparaben | 0,5% |
| | butylenglycol | 0,5% |
| | tocopherole | 0,2% |

### Reference example 17:

### Ointment

An ointment of the oil-in-water-emulsion type, comprising a compound according to formula (I) is prepared from the following ingredients.

| | | |
|---|---|---|
| A | product of formula (I) 10-20% | |
| | butyleneglycol | 5% |
| | glycerol | 4% |
| | sodium dihydroxy cetylphosphate, isopropyl hydroxy cetylether | 2% |
| | water | to 100% |
| | | |
| B | glycolstearate SE | 15% |
| | octylcocoate | 11% |
| | | |
| C | butyleneglycol, metylparabene ethylparabene, propylparabene, pH: adjusted to 5,5 | 2% |

### Literature

Andersen PH, Jensen NJ. Mutagenic investigation of flavourings: dimethyl succinate, ethyl pyruvate and aconitic acid are negative in the Salmonella/mammalian-microsome test. Food Addit Contam. 1984 Jul-Sep;1(3):283-8
Ayoub F, Zaman M, Thornalley P, Masters J. Glyoxalase activities in human tumour cell lines in vitro. Anticancer Res. 1993 Jan-Feb;13(1):151-5.
Bekeredjian R, Graybum PA, Shohet RV. Use of ultrasound contrast agents for gene or drug delivery in cardiovascular medicine. J Am Coll Cardiol. 2005 Feb 1;45(3):329-35.
Bekeredjian R, Chen S, Frenkel PA, Grayburn PA, Shohet RV. Ultrasound-targeted microbubble destruction can repeatedly direct highly specific plasmid expression to the heart. Circulation. 2003 Aug 26;108(8):1022-6. Epub 2003 Aug 11.
Bennett JE, Izumikawa K, Marr KA. Mechanism of increased fluconazole resistance in Candida glabrata during prophylaxis. Antimicrob Agents Chemother. 2004 May;48(5):1773-7.
Birkenmeier G, Heidrich K, Glaser C, Handschug K, Fabricius EM, Frank R, Reissig D. Different expression of the alpha2-macroglobulin receptor/low-density lipoprotein receptor-related protein in human keratinocytes and fibroblasts. Arch Dermatol Res. 1998 Oct;290(10):561-8
Bowmer CT, Hooftman RN, Hanstveit AO, Venderbosch PW, van der Hoeven N. The ecotoxicity and the biodegradability of lactic acid, alkyl lactate esters and lactate salts. Chemosphere. 1998 Sep;37(7):1317-33.
Bozza S, Montagnoli C, Gaziano R, Rossi G, Nkwanyuo G, Bellocchio S, Romani L. Dendritic cell-based vaccination against opportunistic fungi. Vaccine. 2004 Feb 17;22(7):857-64.
Bradford M. M. A rapid and sensitive method for the quantitation of microgram quantities of protein utilizing the principle of protein-dye binding. Anal. Biochem. 1976, 72:248-254.
Brady RL, Cameron A. Structure-based approaches to the development of novel anti-malarials. Curr Drug Targets. 2004 Feb;5(2):137-49.
Carballeira NM, Ortiz D, Parang K, Sardari S Total Synthesis and in vitro-Antifungal Activity of (±)-2-Methoxytetradecanoic Acid Arch. Pharm. Pharm. Med. Chem. 2004 March; 337(3): 152-155.
Clary JJ, Feron VJ, van Velthuijsen JA. Safety assessment of lactate esters. Regul Toxicol Pharmacol. 1998 Apr;27(2):88-97.
Clugston SL, Daub E, Kinach R, Miedema D, Barnard JF, Honek JF. Isolation and sequencing of a gene coding for glyoxalase I activity from Salmonella typhimurium and comparison with other glyoxalase I sequences. Gene. 1997 Feb 20;186(1):103-11.
Creighton DJ, Zheng ZB, Holewinski R, Hamilton DS, Eiseman JL. Glyoxalase I inhibitors in cancer chemotherapy. Biochem Soc Trans. 2003 Dec;31(Pt 6):1378-82.
Cunha BA.Methicillin-resistant Staphylococcus aureus: clinical manifestations and antimicrobial therapy. Clin Microbiol Infect. 2005 Jul;11 Suppl 4:33-42.
Devamanoharan PS, Henein M, Ali AH, Varma SD. Attenuation of sugar cataract by ethyl pyruvate. Mol Cell Biochem. 1999 Oct;200(1-2):103-9.
Doggett MS. Characterization of fungal biofilms within a municipal water distribution system. Appl Environ Microbiol. 2000 Mar;66(3):1249-51
Dong YQ, Yao YM, Wei P, Liu H, Dong N, Yu Y, Sheng ZY. Effects of ethyl pyruvate on cell-mediated immune function in rats with delayed resuscitation after burn injury. Zhongguo Wei Zhong Bing Ji Jiu Yi Xue. 2005 Jan;17(1):12-5.
Douglas KT, Gohel DI, Nadvi IN, Quilter AJ, Seddon AP. Partial transition-state inhibitors of glyoxalase I from human erythrocytes,yeast and rat liver. Biochim Biophys Acta. 1985 May 20;829(1):109-18.
Dufer M, Krippeit-Drews P, Buntinas L, Siemen D, Drews G. Methyl pyruvate stimulates pancreatic beta-cells by a direct effect on KATP channels, and not as a mitochondrial substrate. Biochem J. 2002 Dec 15;368(Pt 3):817-25.
Epstein JB, Hancock PJ, Nantel S. Oral candidiasis in hematopoietic cell transplantation patients: an outcome-based analysis. Oral Surg Oral Med Oral Pathol Oral Radiol Endod. 2003 Aug;96(2):154-63
Fan X, Subramaniam R, Weiss MF, Monnier VM. Methylglyoxal-bovine serum albumin stimulates tumor necrosis factor alpha secretion in RAW 264.7 cells through activation of mitogen-activating protein kinase, nuclear factor kappaB and intracellular reactive oxygen species formation. Arch Biochem Biophys. 2003 Jan 15;409(2):274-86.
Fink MP. Ethyl pyruvate: a novel treatment for sepsis and shock. Minerva Anestesiol. 2004 May;70(5):365-71.
Garcia CK, Goldstein JL, Pathak RK, Anderson RG, Brown MS. Molecular characterization of a membrane transporter for lactate, pyruvate, and other monocarboxylates: implications for the Cori cycle. Cell. 1994 Mar 11;76(5):865-73
Gatenby RA, Gillies RJ. Why do cancers have high aerobic glycolysis? Nat Rev Cancer. 2004 Nov;4(11):891-9.
von Grumbckow L, Eisner P, Hellsten Y, Quistorff B, Juel C.
Kinetics of lactate and pyruvate transport in cultured rat myotubes. Biochim Biophys Acta. 1999 Mar 4;1417(2):267-75.
Gu, J.-D., Ford, T.E., Berke, N.S. & Mitchell, RB Biodeterioration of concrete by the fungus Fusarium. International Biodererioration &Biodegradation. 1998; 41:101-109
Hall S.S., Doweyko L. M., Doweyko A. M., J. S. R.Zilenovski Synthesis and evaluation of alpha-hydroxy-thiol ester as antitumor agents and glyoxalase I inhibitors. J Med Chem. 1977. 20 (10): 1239-1242.
Hamilton D, Batist G. Glutathione analogues in cancer treatment. Curr Oncol Rep. 2004 Mar;6(2):116-22.
Hamilton DS, Creighton DJ. Inhibition of glyoxalase I by the enediol mimic S-(N-hydroxy-N-methylcarbamoyl)glutathione. The possible basis of a tumor-selective anticancer strategy. J Biol Chem. 1992 Dec 15;267(35):24933-6.
Han Y, Englert JA, Yang R, Delude RL, Fink MP. Ethyl Pyruvate Inhibits Nuclear Factor-{kappa}B-Dependent Signaling by Directly Targeting p65. J Pharmacol Exp Ther. 2005 Mar;312(3):1097-105. Epub 2004 Nov 03.
Heymans M, Singh AK. Deriving phylogenetic trees from the similarity analysis of metabolic pathways. Bioinformatics. 2003;19 Suppl 1:i138-46.
Higashiyama Y, Kohno S. Micafungin: a therapeutic review. Expert Rev Anti Infect Ther. 2004 Jun;2(3):345-55.
Iwami Y, Schachtele CF, Yamada T. Mechansim of inhibition of Glycolysis in Streptococcus mutans NCIB 11723 by chlorhexidine. Oral Microbiol. Immunol. 1995 Dec; 10(6):360-4).
de Jaham C. Effects of an ethyl lactate shampoo in conjunction with a systemic antibiotic in the treatment of canine superficial bacterial pyoderma in an open-label, nonplacebo-controlled study. Vet Ther. 2003 Spring;4(1):94-100.
Johansson AS, Ridderstrom M, Mannervik B. The human glutathione transferase P1-1 specific inhibitor TER 117 designed for overcoming cytostatic-drug resistance is also a strong inhibitor of glyoxalase I. Mol Pharmacol. 2000 Mar;57(3):619-24.
Kalsi A, Kavarana MJ, Lu T, Whalen DL, Hamilton DS, Creighton DJ. Role of hydrophobic interactions in binding S-(N-aryl/alkyl-N hydroxycarbamoyl) glutathiones to the active site of the antitumor target enzyme glyoxalase I. J Med Chem. 2000 Oct 19;43(21):3981-6.
Kamiya D, Uchihata Y, Ichikawa E, Kato K, Umezawa K. Reversal of anticancer drug resistance by COTC based on intracellular glutathione and glyoxalase I. Bioorg Med Chem Lett. 2005 Feb 15;15(4):1111-4.
Kavanagh KL, Elling RA, Wilson DK. Structure of Toxoplasma gondii LDH1: active-site differences from human lactate dehydrogenases and the structural basis for efficient APAD+ use. Biochemistry. 2004 Feb 3;43(4):879-89.
Lakhdar-Ghazal F, Blonski C, Willson M, Michels P, Perie J. Glycolysis and proteases as targets for the design of new anti-trypanosome drugs. Curr Top Med Chem. 2002 May;2(5):439-56
Lembert N, Joos HC, Idahl LA, Ammon HP, Wahl MA. Methyl pyruvate initiates membrane depolarization and insulin release by metabolic factors other than ATP. Biochem J. 2001 Mar 1 ;354(Pt 2):345-50
Lluis C, Bozal J. [LDH and structural analogues of pyruvate (author's transl)] Rev Esp Fisiol. 1976 Mar;32(1):9-13
Mannervik B, Aronsson AC, Tibbelin G. Glyoxalase I from human erythrocytes. Methods Enzymol. 1982;90 Pt E:535-41.
Marr KA. Invasive Candida infections: the changing epidemiology. Oncology (Huntingt). 2004 Dec;18(14 Suppl 13):9-14.
Martins AM, Cordeiro C, Freire AP. Glyoxalase II in Saccharomyces cerevisiae: in situ kinetics using the 5,5'-dithiobis(2-nitrobenzoic acid) assay. Arch Biochem Biophys. 1999 Jun 1;366(1):15-20.
Marx E, Mueller-Klieser W, Vaupel P. Lactate-induced inhibition of tumor cell proliferation. Int J Radiat Oncol Biol Phys. 1988 May;14(5):947-55.
McLellan AC, Thornalley PJ. Glyoxalase activity in human red blood cells fractioned by age. Mech Ageing Dev. 1989 Apr;48(1):63-71
Miyaji T, Hu X, Yuen PS, Muramatsu Y, lyer S, Hewitt SM, Star RA. Ethyl pyruvate decreases sepsis-induced acute renal failure and multiple organ damage in aged mice. Kidney Int. 2003 Nov;64(5):1620-31.
Mulier KE, Beilman GJ, Conroy MJ, Taylor JH, Skarda DE, Hammer BE. Ringer's ethyl pyruvate inhemorrhagic shock and resuscitation does not improve early hemodynamics or tissue energetics. Shock. 2005 Mar;23(3):248-252.
Murray MJ, Barbose JJ, Cobb CF. Serum D(-)-lactate levels as a predictor of acute intestinal ischemia in a rat model. J Surg Res. 1993 May;54(5):507-9.
NCCLS National Committee for Clinical Laboratory Standards. Reference method for broth dilution antifungal susceptibility testing of yeast; approved standard. NCCLS document M27-A. Wayne, Pa, 1997
Nesterova M, Cho-Chung YS. Killing the messenger: antisense DNA and siRNA. Curr Drug Targets. 2004 Nov;5(8):683-9.
Pemberton KD, Barrett J. The detoxification of xenobiotic compounds by Onchocerca gutturosa (Nematoda: Filarioidea). Int J Parasitol. 1989 Dec;19(8):875-8.
Perdigon G, Nader de Macias ME, Alvarez S, Oliver G, Pesce de Ruiz Holgado AA. Prevention of gastrointestinal infection using immunobiological methods with milk fermented with Lactobacillus casei and Lactobacillus acidophilus. J Dairy Res. 1990 May;57(2):255-64.
Qin X, Weissman SJ, Chesnut MF, Zhang B, Shen L. Kirby-Bauer disc approximation to detect inducible third-generation cephalosporin resistance in Enterobacteriaceae. Ann Clin Microbiol Antimicrob. 2004 Jul 15;3(1):13.
Randhawa HS. Respiratory and systemic mycoses: an overview. Indian J Chest Dis Allied Sci. 2000 Oct-Dec;42(4):207-19.
Ranganathan S, Walsh ES, Tew KD. Glyoxalase I in detoxification: studies using a glyoxalase I transfectant cell line. Biochem J. 1995 Jul 1;309 (Pt 1):127-31.
Reynolds TB, Fink GR. Bakers' yeast, a model for fungal biofilm formation.Science. 2001 Feb 2;291 (5505):878-81.
Roth DA, Brooks GA. Lactate and pyruvate transport is dominated by a pH gradient-sensitive carrier in rat skeletal muscle sarcolemmal vesicles. Arch Biochem Biophys. 1990 Jun; 279(2):386-94
Sanglard D, Odds FC. Resistance of Candida species to antifungal agents: molecular mechanisms and clinical consequences. Lancet Infect Dis. 2002 Feb;2(2):73-85.
Schwarze R, Seebacher C, Blaschke-Hellmessen R. Candida infections in infancy and early childhood Z Ärztl Fortbild Qualititssich. 1998 Apr;92(3):163-8.
Schwock J, Kirchberger J, Edelmann A, Kriegel TM, Kopperschlager G. Interaction of 6-phosphofructokinase with cytosolic proteins of Saccharomyces cerevisiae. Yeast. 2004 Apr 30;21 (6):483-94.
Sharkey EM, O'Neill HB, Kavarana MJ, Wang H, Creighton DJ, Sentz DL, Eiseman JL. Pharmacokinetics and antitumor properties in tumor-bearing mice of an enediol analogue inhibitor of glyoxalase I. Cancer Chemother Pharmacol. 2000;46(2):156-66.
De Simone C, Tzantzoglou S, Famularo G, Moretti S, Paoletti F, Vullo V, Delia S. High dose L-carnitine improves immunologic and metabolic parameters in AIDS patients. Immunopharmacol Immunotoxicol. 1993 Jan;15(1):1-12.
Stanko RT, Mullick P, Clarke MR, Contis LC, Janosky JE, Ramasastry SS. Pyruvate inhibits growth of mammary adenocarcinoma 13762 in rats. Cancer Res. 1994 Feb 15;54(4):1004-7.
Storgards E, Tapani K, Hartwall P, Saleva R, Suihko M-L Microbial attachment and biofilm formation in brewery bottling plants. J. Am. Soc. Brew. Chem. 2006; 6: 8-15
Sugden MC, Holness MJ. Therapeutic potential of the mammalian pyruvate dehydrogenase kinases in the prevention of hyperglycaemia. Curr Drug Targets Immune Endocr Metabol Disord. 2002 Jul;2(2):151-65.
Szymanska J. Microbiological risk factors in dentistry. Current status of knowledge. Ann Agric Environ Med. 2005;12(2):157-63
Thornalley PJ. Modification of the glyoxalase system in disease processes and prospects for therapeutic strategies. Biochem Soc Trans. 1993 May;21(2):531-4.
Thornalley PJ. Pharmacology of methylglyoxal: formation, modification of proteins and nucleic acids, and enzymatic detoxification-a role in pathogenesis and antiproliferative chemotherapy. Gen Pharmacol. 1996 Jun;27(4):565-73.
Thornalley PJ, Edwards LG, Kang Y, Wyatt C, Davies N, Ladan MJ, Double J. Antitumour activity of S-p-bromobenzylglutathione cyclopentyl diester in vitro and in vivo. Inhibition of glyoxalase I and induction of apoptosis. Biochem Pharmacol. 1996 May 17;51(10):1365-72.
Thornalley PJ, Strath M, Wilson RJ. Antimalarial activity in vitro of the glyoxalase I inhibitor diester, S-p-bromobenzylglutathione diethyl ester. Biochem Pharmacol. 1994 Jan 20;47(2):418-20.
Ulloa L, Ochani M, Yang H, Tanovic M, Halperin D, Yang R, Czura CJ, Fink MP, Tracey KJ. Ethyl pyruvate prevents lethality in mice with established lethal sepsis and systemic inflammation. Proc Natl Acad Sci USA. 2002 Sep 17;99(19):12351-6. Epub 2002 Sep 03
Valverde I, Cancelas J, Villanueva-Penacarrillo ML, Malaisse WJ. Potentiation by methyl pyruvate of GLP-1 insulinotropic action in normal rats. Int J Mol Med. 2001 Jun;7(6):621-3.
Vander Jagt DL, Hunsaker LA, Campos NM, Baack BR. D-lactate production in erythrocytes infected with Plasmodium falciparum. Mol Biochem Parasitol. 1990 Sep-Oct;42(2):277-84.
Varma SD, Devamanoharan PS, Ali AH. Prevention of intracellular oxidative stress to lens by pyruvate and its ester. Free Radio Res. 1998 Feb;28(2):131-5.
Yang R, Uchiyama T, Watkins SK, Han X, Fink MP. Ethyl pyruvate reduces liver injury in a murine model of extrahepatic cholestasis. Shock. 2004 Oct;22(4):369-75.
Vince R, Daluge S. Glyoxalase inhibitors. A possible approach to anticancer agents. J Med Chem. 1971 Jan;14(1):35-7.

### List of abbreviations

- 2-DG: 2-deoxyglucose
- AIDS: Aquired immunodeficiency syndrom
- ATP: Adenosine triphosphate
- ATCC: American Type Culture Collection
- BBB: Blood Brain Barrier
- BL: butyl lactate
- BP: butyl pyruvate
- CFU: Colony forming unit
- CNS: Central nervous system
- CSF: colony stimulation factor
- DBL: butyl D-lactate
- DEL: Ethyl D-lactate
- DMEM: Dulbecco's modified Eagle Medium
- DMSO: dimethyl sulfoxide
- DSMZ: German Collection of Microorganisms and Cell Cultures GmbH (Deutsche Sammlung von Mikroorganismen und Zellkulturen GmbH)
- DTNB: 5,5'-dithiobis(2-nitro benzoic acid)
- E.C.: Enzyme Commission
- EDTA: ethylene diamine tetraacetate
- EOB: ethyl -2-oxo-butyrate
- EP: ethyl pyruvate
- FGF: fibroblast growth factor
- FCS: fetal calf serum
- GLO: I Glyoxalase 1
- HEPES: 4-(2-hydroxyethyl)-1-piperazineethanesulfonic acid
- IG50: Inhibitor concentration to reach 50% of the maximal effect
- IU: international unit
- LBL: Butyl L-lactate
- LEL: Ethyl L-lactate
- MES: 4-morpholine ethanesulfonic acid
- MCT: monocarboxylate transporter
- MIC: Minimal inhibition concentration
- MRSA: Methicillin-resistant Staphylococcus Aureus
- MSSA: Methicillin-sensitive Staphylococcus Aureus
- NFkB: nuclear factor kappa B
- INF: interferon
- O.D.: optical density
- PBS: phosphate buffered saline
- RPMI: Rosswell Park Memorial Institute
- SF: serum free
- YPD: yeast peptone dextrose medium

## Claims

1. Pharmaceutical composition for topic administration comprising
at least two pharmaceutically active compounds
wherein one pharmaceutically active compound is a substance according to the general formula (I) as antifungal agent, wherein said substance according to formula (I) is present in a therapeutically effective concentration: 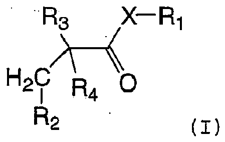 wherein
X is O or S; and
R1 is a branched or non-branched alkyl, cycloalkyl, branched or non-branched alkenyl, cycloalkenyl, branched or non-branched alkinyl, cycloalkinyl, alkoxyalkyl, alkoxycarbonylalkyl, aryl or a sugar residue; and
R2 is H or a branched or non-branched alkyl, cycloalkyl, branched or non-branched alkenyl, cycloalkenyl, branched or non-branched alkinyl, cycloalkinyl, alkoxyalkyl, alkoxycarbonylalkyl or aryl residue; and
R3 and R4 together are =0; or R3 is OH and R4 is H; or R3 is H and R4 is OH,
with the proviso that when X is O, R2 is H and R3 or R4 is OH, then R1 is not C1-C12 alkyl,
for use in the treatment and/or prophylaxis of a fungal infection in an animal, including humans, wherein the second pharmaceutically active compound is glucose.

2. The pharmaceutical composition according to claim 1, wherein R1 comprises 1 to 8 carbon atoms and R2 is H or comprises 1 to 8 carbon atoms.

3. The pharmaceutical composition according to anyone of claims 1 or 2, wherein R1 comprises 1 to 4 carbon atoms and R2 is H or comprises 1 to 4 carbon atoms.

4. The pharmaceutical composition according to any one of claims 1 to 3, wherein R1, R2, or R1 and R2 are methyl, ethyl, propyl, isopropyl, butyl, or isobutyl.

5. The pharmaceutical composition according to any one of claims 1 to 4, wherein said substance according to formula (I) is one or more selected from methyl pyruvate, ethyl pyruvate, propyl pyruvate, isopropyl pyruvate, butyl pyruvate, isobutyl pyruvate, ethyl 2-oxobutyrate, butyl-2-oxo-butyrate, cyclohexylmethyl pyruvate or the said compounds wherein X = S.

6. The pharmaceutical composition according to any one of claims 1 to 5, wherein in said substance R3 or R4 is OH, and it is selected from the group comprising the D-, L-enantiomer and the racemic mixture thereof.

7. The pharmaceutical composition according to any one of claims 1 to 6, which comprises one or more additional pharmaceutically active ingredients.

8. The pharmaceutical composition according to claim 7, wherein an additional pharmaceutically active ingredient is selected from antifungal agents.

9. The pharmaceutical composition according to claim 8, wherein said antifungal agent is one or more selected from fluconazole, ketoconazole, clotrimazole, bifonazole, econazole, itraconazole, terbinafine, naftifine, amorolfine, amphotericin B, nystatin, and nikkomycin, miconazole, oxiconazole, sulconazole, flucytosine, griseofulvin, and biocidal peptides including defensins.

10. The pharmaceutical composition according to any one of claims 1 to 9, which further comprises one or more auxiliary substances, comprising fillers, flavouring agents and stabilizers.

11. The pharmaceutical composition according to any one of claims 1 to 10, wherein said composition is in the form of a sustained release or controlled release galenic formulation.

12. The pharmaceutical composition according to any one of claims 1 to 11, which is for one or more selected from the group comprising intraauricular, intranasal, percutaneous, transdermal, pulmonary administration, or administration as an aerosol, as mouth lavage, as an oil, cream, ointment, spray, gel, and plaster.

13. The pharmaceutical composition according to any one of claims 1 to 12, wherein said animal is selected from the group comprising invertebrates, non-mammalian vertebrates, and mammals including humans.

14. The pharmaceutical composition according to any one of claims 1 to 13, which is for the treatment, prophylaxis, or treatment and prophylaxis of fungal infections resistant to antibiotic treatment.

15. The pharmaceutical composition according to claim 14, wherein said resistance is to one or more selected from the group comprising fluconazole, ketoconazole, clotrimazole, bifonazole, econazole, itraconazole, terbinafine, naftifine, amorolfine, amphotericin B, nystatin, and nikkomycin, miconazole, oxiconazole, sulconazole, flucytosine, griseofulvin, and biocidal peptides including defensins.

16. The pharmaceutical composition according to any one of claims 1 to 14, which is for the treatment of infections of one or more of the genus Candida., Aspergillus, Cryptococcus, Pneumocystis, Zygomyces, Dermatophytes, Blastomyces, Histoplasma, Coccidoides, Sporothrix, Microsporidia, Malassezia and Basidiomycetes.

17. The pharmaceutical composition according to any one of claims 1 to 16, wherein said fungal infection is an opportunistic infection.

18. The pharmaceutical composition according to any one of claims 1 to 17, which is for use in an immunosuppressed animal including man.

19. The pharmaceutical composition according to claim 18, wherein said immunosuppression is associated with one or more selected from hereditary and acquired immune-defects.

20. The pharmaceutical composition according to claim 19, wherein said acquired immune defect is associated with one or more selected from the group comprising HIV, organ transplantation, chemotherapy and exposure to radiation.

21. The pharmaceutical composition according to any one of claims 1 to 20, which is for use in an animal, including man, concomitantly suffering from one or more selected from bacterial and protozoal infection.

22. The pharmaceutical composition according to any one of claims 1 to 21, which is for use in an animal, including man, having a reduced blood glucose level.

23. The pharmaceutical composition according to any one of claims 1 to 22, which is for use in an animal, including man, concomitantly suffering from cancer.

24. The pharmaceutical composition according to any one of claims 1 to 23, wherein said animal, including man, is going to receive, is currently receiving, or has received conventional cancer therapy.

25. The pharmaceutical composition according to claim 24, wherein said conventional cancer therapy is one or more of chemotherapy, surgery, radiotherapy or brachytherapy.

## Patentansprüche

1. Pharmazeutische Zusammensetzung zur topischen Verabreichung, Folgendes umfassend:
wenigstens zwei pharmazeutische Wirkstoffe
wobei ein pharmazeutischer Wirkstoff eine Substanz nach der allgemeinen Formel (I) als antifungales Mittel ist, wobei die Substanz nach der Formel (I) in einer therapeutisch wirksamen Konzentration vorliegt: 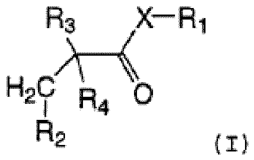
wobei
X O oder S ist; und
R1 ein verzweigtes oder nichtverzweigtes Alkyl, Cycloalkyl, verzweigtes oder nichtverzweigtes Alkenyl, Cycloalkenyl, verzweigtes oder nichtverzweigtes Alkinyl, Cycloalkinyl, Alkoxyalkyl, Alkoxycarbonylalkyl, Aryl oder ein Zuckerrest ist; und
R2 H oder ein verzweigtes oder nichtverzweigtes Alkyl, Cycloalkyl, verzweigtes oder nichtverzweigtes Alkenyl, Cycloalkenyl, verzweigtes oder nichtverzweigtes Alkinyl, Cycloalkinyl, Alkoxyalkyl, Alkoxycarbonylalkyl oder ein Arylrest ist; und
R3 und R4 beide =0 sind; oder R3 OH ist und R4 H ist; oder R3 H ist und R4 OH ist, unter der Bedingung, dass, wenn X O ist, R2 H ist und R3 oder R4 OH ist, dann R1 nicht C1-C12-Alkyl ist,
für den Gebrauch bei der Behandlung einer und / oder bei der Prophylaxe gegen eine Pilzinfektion in einem Tier, einschließlich Menschen, wobei der zweite pharmazeutische Wirkstoff Glucose ist.

2. Pharmazeutische Zusammensetzung nach Anspruch 1, wobei R1 1 bis 8 Kohlenstoffatome umfasst und R2 H ist oder 1 bis 8 Kohlenstoffatome umfasst.

3. Pharmazeutische Zusammensetzung nach einem der Ansprüche 1 oder 2, wobei R1 1 bis 4 Kohlenstoffatome umfasst und R2 H ist oder 1 bis 4 Kohlenstoffatome umfasst.

4. Pharmazeutische Zusammensetzung nach einem der Ansprüche 1 bis 3, wobei R1, R2 oder sowohl R1 als auch R2 Methyl, Ethyl, Propyl, Isopropyl, Butyl oder Isobutyl sind.

5. Pharmazeutische Zusammensetzung nach einem der Ansprüche 1 bis 4, wobei die Substanz nach der Formel (I) eine oder mehrere der Folgenden ist: Methylpyruvat, Ethylpyruvat, Propylpyruvat, Isopropylpyruvat, Butylpyruvat, Isobutylpyruvat, Ethyl-2-oxobutyrat, Butyl-2-oxo-butyrat, Cyclohexylmethylpyruvat oder die Verbindungen, wobei X S ist.

6. Pharmazeutische Zusammensetzung nach einem der Ansprüche 1 bis 5, wobei in der Substanz R3 oder R4 OH ist, und die ausgewählt ist aus der Gruppe bestehend aus dem D-, L-Enantiomer und der racemischen Mischung daraus.

7. Pharmazeutische Zusammensetzung nach einem der Ansprüche 1 bis 6, die einen oder mehrere zusätzlicher pharmazeutische Wirkstoffe umfasst.

8. Pharmazeutische Zusammensetzung nach Anspruch 7, wobei ein zusätzlicher pharmazeutischer Wirkstoff aus der Gruppe der antifungalen Mittel ausgewählt ist.

9. Pharmazeutische Zusammensetzung nach Anspruch 8, wobei das antifungale Mittel eines oder mehrere der Folgenden ist: Fluconazol, Ketoconazol, Clotrimazol, Bifonazol, Econazol, Itraconazol, Terbinafin, Naftifin, Amorolfin, Amphotericin B, Nystatin und Nikkomycin, Miconazol, Oxiconazol, Sulconazol, Flucytosin, Griseofulvin und biozide Peptide, einschließlich Defensine.

10. Pharmazeutische Zusammensetzung nach einem der Ansprüche 1 bis 9, die ferner eine oder mehrere zusätzliche Substanzen umfasst, umfassend Füllstoffe, Aromastoffe und Stabilisatoren.

11. Pharmazeutische Zusammensetzung nach einem der Ansprüche 1 bis 10, wobei die Zusammensetzung in der Form einer galenischen Formulierung zur verzögerten Freigabe oder gesteuerter Freigabe vorliegt.

12. Pharmazeutische Zusammensetzung nach einem der Ansprüche 1 bis 11, für eine oder mehrere der Verabreichungsformen, ausgewählt aus der Gruppe bestehend aus: intraaurikular, intranasal, perkutan, transdermal, pulmonar, oder Verabreichung als Aerosol, als Mundspülung, als Öl, Creme, Salbe, Spray, Gel oder Pflaster.

13. Pharmazeutische Zusammensetzung nach einem der Ansprüche 1 bis 12, wobei das Tier aus der Gruppe ausgewählt ist, die aus Wirbellosen, Nichtsäuger-Wirbeltieren und Säugern, einschließlich Menschen, besteht.

14. Pharmazeutische Zusammensetzung nach einem der Ansprüche 1 bis 13 für die Behandlung einer und / oder die Prophylaxe gegen eine Pilzinfektion, die gegen antibiotische Behandlung resistent ist.

15. Pharmazeutische Zusammensetzung nach Anspruch 14, wobei die Resistenz gegen Stoffe ausgewählt ist aus der Gruppe bestehend aus Fluconazol, Ketoconazol, Clotrimazol, Bifonazol, Econazol, Itraconazol, Terbinafin, Naftifin, Amorolfin, Amphotericin B, Nystatin und Nikkomycin, Miconazol, Oxiconazol, Sulconazol, Flucytosin, Griseofulvin und bioziden Peptiden, einschließlich Defensinen.

16. Pharmazeutische Zusammensetzung nach einem der Ansprüche 1 bis 14, zur Behandlung von Infektionen von einem oder mehreren der Gattungen Candida, Aspergillus, Cryptococcus, Pneumocystis, Zygomyces, Dermatophytes, Blastomyces, Histoplasma, Coccidoides, Sporothrix, Microsporidia, Malassezia und Basidiomycetes.

17. Pharmazeutische Zusammensetzung nach einem der Ansprüche 1 bis 16, wobei die Pilzinfektion eine opportunistische Infektion ist.

18. Pharmazeutische Zusammensetzung nach einem der Ansprüche 1 bis 17, zum Gebrauch in einem immunsupprimierten Tier, einschließlich Menschen.

19. Pharmazeutische Zusammensetzung nach Anspruch 18, wobei die Immunsupprimierung mit vererbten und / oder erworbenen Immundefekten verbunden ist.

20. Pharmazeutische Zusammensetzung nach Anspruch 19, wobei der erworbene Immundefekt mit einem oder mehreren aus der Gruppe bestehend aus HIV, Organtransplantation, Chemotherapie und Strahlenaussetzung verbunden ist.

21. Pharmazeutische Zusammensetzung nach einem der Ansprüche 1 bis 20 zum Gebrauch in einem Tier, einschließlich Menschen, der gleichzeitig an einer bakteriellen und / oder protozoalen Infektion leidet.

22. Pharmazeutische Zusammensetzung nach einem der Ansprüche 1 bis 21, zum Gebrauch in einem Tier, einschließlich Menschen, mit einem verminderten Blutzuckerspiegel.

23. Pharmazeutische Zusammensetzung nach einem der Ansprüche 1 bis 22, zum Gebrauch in einem Tier, einschließlich Menschen, das / der gleichzeitig an Krebs leidet.

24. Pharmazeutische Zusammensetzung nach einem der Ansprüche 1 bis 23, wobei das Tier, einschließlich Menschen, eine herkömmliche Krebstherapie erhalten wird, gegenwärtig erhält oder zuvor erhalten hat.

25. Pharmazeutische Zusammensetzung nach Anspruch 24, wobei die herkömmliche Krebstherapie eine oder mehrere der Folgenden ist: Chemotherapie, Operation, Strahlentherapie oder Brachytherapie.

## Revendications

1. Composition pharmaceutique pour une administration topique comprenant
au moins deux composés pharmaceutiquement actifs
où un composé pharmaceutiquement actif est une substance répondant à la formule générale (I) en tant qu'agent antifongique, ladite substance répondant à la formule (I) étant présente à une concentration efficace sur le plan thérapeutique : 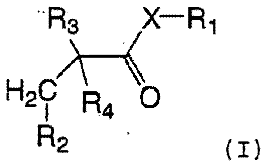 dans laquelle
X représente O ou S ; et
R1 représente un résidu alkyle ramifié ou non ramifié, cycloalkyle, alcényle ramifié ou non ramifié, cycloalcényle, alkynyle ramifié ou non ramifié, cycloalkinyle, alkoxyalkyle, alkoxycarbonylalkyle, aryle ou osidique ; et
R2 représente un H ou un résidu alkyle ramifié ou non ramifié, cycloalkyle, alcényle ramifié ou non ramifié, cycloalcényle, alkynyle ramifié ou non ramifié, cycloalkinyle, alkoxyalkyle, alkoxycarbonylalkyle ou aryle ; et
R3 et R4 forment ensemble un =0 ; ou R3 représente un OH et R4 représente un H ; ou R3 représente un H et R4 représente un OH,
à la condition que quand X représente un O, R2 représente un H et R3 ou R4 représente un OH, R1 ne représente pas un alkyle en C1-C12,
pour une utilisation dans le traitement et/ou la prophylaxie d'une infection fongique chez un animal, y compris un humain, le deuxième composé pharmaceutiquement actif étant le glucose.

2. Composition pharmaceutique selon la revendication 1, dans laquelle R1 comprend de 1 à 8 atomes de carbone et R2 représente un H ou comprend de 1 à 8 atomes de carbone.

3. Composition pharmaceutique selon l'une quelconque des revendications 1 ou 2, dans laquelle R1 comprend de 1 à 4 atomes de carbone et R2 représente un H ou comprend de 1 à 4 atomes de carbone.

4. Composition pharmaceutique selon l'une quelconque des revendications 1 à 3, où R1, R2, ou R1 et R2, représentent un méthyle, éthyle, propyle, isopropyle, butyle ou isobutyle.

5. Composition pharmaceutique selon l'une quelconque des revendications 1 à 4, dans laquelle ladite substance répondant à la formule (I) est une ou plusieurs de celles sélectionnées parmi le pyruvate de méthyle, le pyruvate d'éthyle, le pyruvate de propyle, le pyruvate d'isopropyle, le pyruvate de butyle, le pyruvate d'isobutyle, le 2-oxobutyrate d'éthyle, le 2-oxobutyrate de butyle, le pyruvate de cyclohexylméthyle ou lesdits composés dans lesquels X = S.

6. Composition pharmaceutique selon l'une quelconque des revendications 1 à 5, dans laquelle, dans ladite substance R3 ou R4 représente un OH et est sélectionnée dans le groupe comprenant l'énantiomère D, l'énantiomère L et le mélange racémique de ceux-ci.

7. Composition pharmaceutique selon l'une quelconque des revendications 1 à 6, qui comprend un ou plusieurs ingrédients pharmaceutiquement actifs supplémentaires.

8. Composition pharmaceutique selon la revendication 7, dans laquelle l'ingrédient pharmaceutiquement actif supplémentaire est sélectionné parmi des agents antifongiques.

9. Composition pharmaceutique selon la revendication 8, dans laquelle ledit agent antifongique est un ou plusieurs de ceux sélectionnés parmi les suivants : fluconazole, kétoconazole, clotrimazole, bifonazole, éconazole, itraconazole, terbinafine, naftifine, amorolfine, amphotéricine B, nystatine et nikkomycine, miconazole, oxiconazole, sulconazole, flucytosine, griséofulvine et peptides biocides, y compris des défensines.

10. Composition pharmaceutique selon l'une quelconque des revendications 1 à 9, qui comprend en outre une ou plusieurs substances auxiliaires incluant des matières de charge, des aromatisants et des stabilisants.

11. Composition pharmaceutique selon l'une quelconque des revendications 1 à 10, ladite composition étant sous la forme d'une préparation galénique à libération prolongée ou à libération contrôlée.

12. Composition pharmaceutique selon l'une quelconque des revendications 1 à 11, qui est conçue pour être utilisée par une ou plusieurs voies sélectionnées parmi les suivantes : administration intra-auriculaire, intranasale, percutanée, transdermique, pulmonaire ou administration en aérosol, sous la forme d'un bain de bouche, d'une huile, d'une crème, d'une pommade, d'une préparation pulvérisée, d'un gel et d'un pansement.

13. Composition pharmaceutique selon l'une quelconque des revendications 1 à 12, dans laquelle ledit animal est sélectionné dans le groupe comprenant des invertébrés, des vertébrés non mammifères et des mammifères, y compris des humains.

14. Composition pharmaceutique selon l'une quelconque des revendications 1 à 13 qui est indiquée dans le traitement, la prophylaxie ou le traitement et la prophylaxie d'infections fongiques résistantes à une antibiothérapie.

15. Composition pharmaceutique selon la revendication 14, dans laquelle ladite résistance est envers un ou plusieurs composés sélectionnés parmi les suivants : fluconazole, kétoconazole, clotrimazole, bifonazole, éconazole, itraconazole, terbinafine, naftifine, amorolfine, amphotéricine B, nystatine et nikkomycine, miconazole, oxiconazole, sulconazole, flucytosine, griséofulvine et peptides biocides, y compris des défensines.

16. Composition pharmaceutique selon l'une quelconque des revendications 1 à 14, qui est indiquée dans le traitement d'infections par un ou plusieurs pathogènes des genres ou classes suivants : Candida, Aspergillus, Cryptococcus, Pneumocystis, zygomycètes, dermatophytes, Blastomyces, Histoplasma, Coccidioides, Sporotrichum, microsporidies, Malassezia et basidiomycètes.

17. Composition pharmaceutique selon l'une quelconque des revendications 1 à 16, ladite infection fongique étant une infection opportuniste.

18. Composition pharmaceutique selon l'une quelconque des revendications 1 à 17, qui est indiquée pour une utilisation chez un animal, y compris un humain, qui présente une immunosuppression.

19. Composition pharmaceutique selon la revendication 18, ladite immunosuppression étant associée à un ou plusieurs troubles sélectionnés parmi des déficits immunitaires héréditaires et acquis.

20. Composition pharmaceutique selon la revendication 19, où ledit déficit immunitaire acquis est associé à une ou plusieurs causes sélectionnées dans le groupe comprenant une infection à VIH, la transplantation d'un organe, une chimiothérapie et une exposition à des radiations.

21. Composition pharmaceutique selon l'une quelconque des revendications 1 à 20, qui est indiquée pour une utilisation chez un animal, y compris un humain, souffrant également d'une ou de plusieurs maladies sélectionnées parmi une infection bactérienne et une protozoose.

22. Composition pharmaceutique selon l'une quelconque des revendications 1 à 21, qui est indiquée pour une utilisation chez un animal, y compris un humain, chez lequel la glycémie est basse.

23. Composition pharmaceutique selon l'une quelconque des revendications 1 à 22, qui est indiquée pour une utilisation chez un animal, y compris un humain, souffrant également d'un cancer.

24. Composition pharmaceutique selon l'une quelconque des revendications 1 à 23, où ledit animal, y compris ledit humain, va recevoir, reçoit actuellement ou a reçu une thérapie anticancéreuse conventionnelle.

25. Composition pharmaceutique selon la revendication 24, où ladite thérapie anticancéreuse conventionnelle est un ou plusieurs des suivantes : chimiothérapie, chirurgie, radiothérapie ou curiethérapie.
